# EUROPEAN PATENT APPLICATION

(11) **EP 2 087 789 A1**
(43) Date of publication of application: **12.08.2009**
(21) Application number: 08151122.2
(22) Date of filing: 06.02.2008
(51) Int. Cl.: A01K 67/027, C12N 5/00, G01N 33/50, C07K 16/40, C12N 15/53

(54) **Fto-modified non-human mammal**

(71) Applicant: Heinrich-Heine-Universität Düsseldorf, 40225 Düsseldorf (DE)
(72) Inventor: Rüther, Ulrich, 51381 Leverkusen (DE); Fischer, Julia, 45257 Essen (DE); Emmerling, Christian, 40225 Düsseldorf (DE)
(74) Representative: Michalski Hüttermann & Partner Patentanwälte

(57) **Abstract**

The present invention relates to a non-human mammal being partially or completely deficient in expressing a functional gene product encoded by the *Fto* gene, as well as to a non-human mammal, said mammal being **characterized in that** the expression level of an Fto protein is increased.

## Description

### Field of the invention

The present invention relates generally to the field of obesity. More particularly, it concerns the identification of genes and proteins responsible for obesity and to their inhibitors for use in therapeutics.

### Background of the invention

Obesity is increasingly viewed as a serious and growing public health problem. Especially morbid obesity is a serious disease process, in which the accumulation of fatty tissue on the body becomes excessive, interferes with, or injures the other bodily organs, and creates or predictably will create serious and life-threatening health problems, which are called co-morbidities. It is said that people are morbidly obese when their Body Mass Index (BMI =weight (kg) / (height (m))² ) is equal to or over 40.

Obesity has been shown to predispose to various diseases. Obesity associated diseases comprise cardiovascular diseases and type II diabetes.

Numerous scientific studies have established that there is a genetic predisposition to obesity and morbid obesity, respectively. Thus, genetic predisposition is an object of numerous studies.

Reed et al. postulate that numerous genes are predicted to influence the body weight of a mouse. If it is assumed that the knock-out genes they surveyed are representative then upward of 6,000 genes are predicted to be involved in obesity (Reed et al., 2008)¹.

A region of the chromosome 10 has been identified as being involved in obesity (Hager et al., 1998) ², which has been confirmed in a cohort of German young obese subjects (Hinney et al., 2000) ³, as well as in White Caucasians and in African Americans (Price et al., 2001) ⁴.

EP 1 428 875 B1 relates to three new identified single nucleotide polymorphisms (SNPs), which are located in said region of the chromosome 10, and more particularly in the 5' region of the *gad2* gene, wherein said alterations are indicative of a predisposition to obesity.

The fat mass and obesity associated (*FTO*) gene is a gene on human chromosome 16.

FTO contains a nuclear localization signal and shows a ubiquitous expression pattern including metabolically relevant tissue such as pancreas, liver and the hypothalamus (Gerken et al., 2007) ⁵. The physiologically relevant substrate of FTO, as well as its functional relationship to the development of obesity, however, remain undetermined. Moreover, the functional role of FTO in energy homeostasis remains elusive.

### Definitions

Unless defined otherwise, technical and scientific terms used herein have the same meaning as commonly understood by one of ordinary skill in the art to which this invention belongs.

Terms denoting genes, as used herein, are represented in italics. Terms denoting genes or proteins of human origin, as used herein, are represented in capitals. The term *"Fto* gene", as used herein, is meant to refer to non-human mammal, in particular mouse, genetic material which expresses "Fto proteins". The term "Fto proteins", as used herein, is meant to refer to non-human mammal, in particular mouse, Fto proteins. The term *"FTO* gene", as used herein, is meant to refer to human genetic material which expresses "FTO proteins". The term "FTO proteins", as used herein, is meant to refer to human FTO proteins.

The term "functional gene product", as used herein, is meant to refer to the biochemical activity of a gene product.

The term "deficient in expressing", as used herein, is meant to refer to a decreased expression level compared to wildtype expression level. This deficiency can be only partial or complete.

The term "non-human knock-out mammal" as used herein, is meant to refer to a non-human mammal being partially or completely deficient in expressing a functional gene product encoded by the *Fto* gene.

The term "mutation", as used herein, is meant to refer to changes to the base pair sequence of the genetic material of an organism.

The term "transgene", as used herein, is meant to refer to a gene or transgenic material that has been transferred by any of a number of genetic engineering techniques from one organism to another.

The term "small molecule drug", as used herein, is meant to refer to a medicinal drug compound having a molecular weight of less than 1000 Daltons, and typically between 300 and 700 Daltons. Small molecules can be nucleic acids, peptides, polypeptides, peptidomimetics, carbohydrates, lipids or other organic carbon-containing or inorganic molecules. Small molecule drugs can target enzyme- or receptor-binding sites to inhibit disease-causing proteins.

The term "expression level", as used herein, is meant to refer to the process by which a gene's DNA sequence is converted into functional protein and particularly to the amount of said conversion. Alteration of mRNA expression can be detected by any techniques known in the art. These include Northern blot analysis, PCR amplification and RNase protection. In one embodiment, mRNA of the sample is contacted with a *Fto* gene probe under conditions suitable for hybridization of said probe to a RNA corresponding to said *Fto* gene and hybridization of said probe is determined, and the level of signal after hybridization is compared with a standard (reference) signal. The hybridization complex emits a signal, which may be due to the labeling of the probe, or of the mRNA. The different labels that may be used are well known to the person skilled in the art, and one can cite ³²P, ³³P, ³⁵S, ³H or ¹²⁵I. Non radioactive labels may be selected from ligands as biotin, avidin, streptavidin, dioxygenin, haptens, dyes, luminescent agents like radioluminescent, chemoluminescent, bioluminescent, fluorescent or phosphorescent agents.

The term "yeast two-hybrid system", as used herein, is meant to refer to an assay used for the identification of protein interaction partners. In the two-hybrid system, two fusion proteins are created: the protein of interest (X, or prey), which is constructed to have a DNA binding domain attached to its N-terminus, and its potential binding partner (Y, or bait), which is fused to an activation domain. If protein X interacts with protein Y, the binding of these two will form an intact and functional transcriptional activator. This newly formed transcriptional activator will then go on to transcribe a reporter gene, which is simply a gene whose protein product can be easily detected and measured. In this way, the amount of the reporter produced can be used as a measure of interaction between the protein of interest and its potential partner

The term "GST pull-down assay", as used herein, is meant to refer to an assay used for the identification of protein interaction partners. GST pull-down assay is a technique to test interaction between a tagged protein or the bait (GST, His6, biotin ...) and another protein (test protein, or prey). The bait protein, purified from an appropriate expression system (e.g., *Escherichia coli*), is immobilized on a glutathione affinity gel. The bait serves as the secondary affinity support for identifying new protein partners or for confirming a previously suspected protein partner to the bait. Prey protein can be obtained from multiple sources including recombinant purified proteins, cell lysate or *in vitro* transcription/translation reactions. Protein-protein interactions can be visualized by SDS-PAGE and associated detection methods depending on the sensitivity requirements of the interacting proteins. These methods include Coomassie or silver staining, Western blotting and [35S] radioisotopic detection.

The term "transposons", as used herein, is meant to refer to sequences of DNA that can move around to different positions within the genome of a single cell, a process called transposition. In the process, they can cause mutations and change the amount of DNA in the genome. Transposons are also called "jumping genes", and are examples of mobile genetic elements.

The term "site directed mutagenesis", as used herein, is meant to refer to a molecular biology technique in which a mutation is created at a defined site in a DNA molecule, usually a circular molecule known as a plasmid (Ling MM and Robinson BH, "Approaches to DNA mutagenesis: an overview", Anal. Biochem. 1997; 254: 157-178). In general, site-directed mutagenesis requires that the wild-type gene sequence be known. This technique is also known as site-specific mutagenesis or Oligonucleotide-directed mutagenesis.

The term "gene targeting", as used herein, is meant to refer to a genetic technique that uses homologous recombination to change an endogenous gene (Joyner AL, "Gene Targeting: A Practical Approach", IRL Press 1993). The method can be used to delete a gene, remove exons, and introduce point mutations. Gene targeting can be permanent or conditional. Conditions can be a specific time during development / life of the organism or limitation to a specific tissue, for example. It can be used for any gene, regardless of transcriptional activity or gene size.

The term "mutagen", as used herein, is meant to refer to a physical or chemical agent that changes the genetic information (usually DNA) of an organism and thus increases the frequency of mutations above the natural background level. As many mutations cause cancer, mutagens are typically also carcinogens. Examples for mutagens are ionizing radiation, such as ultraviolet light, gamma rays and alpha particles, intercalating agents, such as ethidium bromide and alkylating agents, such as bromouracil.

The term "antisense RNA" and/or "antisense construct", as used herein, is meant to refer to single-stranded RNA that is complementary to a messenger RNA (mRNA) strand transcribed within a cell. Antisense RNA and antisense construct, respectively, may be introduced into a cell to inhibit translation of a complementary mRNA by base pairing to it and physically obstructing the translation machinery.

The term "RNA interference" (RNAi), as used herein, is meant to refer to a mechanism that inhibits gene expression by causing the degradation of specific RNA molecules or hindering the transcription of specific genes. RNAi targets include RNA from viruses and transposons (probably as a form of innate immune response), and also plays a role in regulating development and genome maintenance. Small interfering RNA strands (siRNA) are key to the RNAi process, and have complementary nucleotide sequences to the targeted RNA strand. Specific RNAi pathway proteins are guided by the siRNA to the targeted messenger RNA (mRNA), where they "cleave" the target, breaking it down into smaller portions that can no longer be translated into protein. A type of RNA transcribed from the genome itself, microRNA (miRNA), works in the same way. The RNAi pathway is initiated by the enzyme dicer, which cleaves long, double-stranded RNA (dsRNA) molecules into short fragments of 20-25 base pairs. One of the two strands of each fragment, known as the guide strand, is then incorporated into the RNA-induced silencing complex (RISC) and pairs with complementary sequences. The most well-studied outcome of this recognition event is post-transcriptional gene silencing. This occurs when the guide strand specifically pairs with a mRNA molecule and induces the degradation by argonaute, the catalytic component of the RISC complex. Another outcome is epigenetic changes to a gene - histone modification and DNA methylation - affecting the degree the gene is transcribed. The selective and robust effect of RNAi on gene expression makes it a valuable research tool, both in cell culture and in living organisms because synthetic dsRNA introduced into cells can induce suppression of specific genes of interest. RNAi may also be used for large-scale screens that systematically shut down each gene in the cell, which can help identify the components necessary for a particular cellular process or an event such as cell division.

The term "interaction", as used herein, is meant to refer to a kind of action that occurs as two more objects have an effect upon one another. The interactions between proteins are important for many biological functions. For example, signals from the exterior of a cell are mediated to the inside of that cell by protein-protein interactions of the signaling molecules. This process, called signal transduction, plays a fundamental role in many biological processes and in many diseases. Proteins might interact for a long time to form part of a protein complex, a protein may be carrying another protein (for example, from cytoplasm to nucleus or vice versa in the case of the nuclear pore importins), or a protein may interact briefly with another protein just to modify it (for example, a protein kinase will add a phosphate to a target protein). This modification of proteins can itself change protein-protein interactions. For example, some proteins with Src homology 2 (SH2) domains only bind to other proteins when they are phosphorylated on the amino acid tyrosine. The term "interaction partner", as used herein, is meant to refer to a protein described above having an effect upon another protein.

The term "fragment of the nucleic acid molecule" is intended to indicate a nucleic acid comprising a subset of a nucleic acid molecule according to one of the claimed sequences. The same is applicable to the term "fraction of the nucleic acid molecule".

The term "variant of the nucleic acid molecule" refers herein to a nucleic acid molecule which is substantially similar in structure and biological activity to a nucleic acid molecule according to one of the claimed sequences. Preferably, said term refers to a nucleic acid molecule which comprises at least one silent single nucleotide mutation (as allowed by the degeneracy of the genetic code).

The term "homologue of the nucleic acid molecule" refers to a nucleic acid molecule the sequence of which has one or more nucleotides added, deleted, substituted or otherwise chemically modified in comparison to a nucleic acid molecule according to one of the claimed sequences, provided always that the homologue retains substantially the same binding properties as the latter.

The term "derivative," as used herein, refers to a nucleic acid molecule that has similar binding characteristics to a target nucleic acid sequence as a nucleic acid molecule according to one of the claimed sequences

The term "directed evolution approach," as used herein, refers to a random mutagenesis. The random mutagenesis is applied to a protein, and a selection regime is used to pick out variants that have the desired qualities. Further rounds of mutation and selection are then applied. This method mimics natural evolution and generally produces superior results to rational design. An additional technique known as DNA shuffling mixes and matches pieces of successful variants in order to produce better results. This process mimics recombination that occurs naturally during sexual reproduction. The great advantage of directed evolution techniques is that they require no prior structural knowledge of a protein, nor it is necessary to be able to predict what effect a given mutation will have. Indeed, the results of directed evolution experiments are often surprising in that desired changes are often caused by mutations that no one would have expected.

### Object of the invention

An object of the present invention is to provide a non-human mammal model being useful as an experimental animal for the elucidation of the physiological role of Fto.

This object is met with means according to the independent claims of the present invention. The dependent claims are related to preferred embodiments. It is yet to be understood that value ranges delimited by numerical values are to be understood to include the said delimiting values.

### Summary of the invention

Before the invention is described in detail, it is to be understood that this invention is not limited to the particular biological systems. The described biological systems may vary. It is also to be understood that the terminology used herein is for purposes of describing particular embodiments only, and is not intended to be limiting. It must be noted that, as used in the specification and the appended claims, the singular forms "a", "an" and "the" include singular and/or plural referents unless the context clearly dictates otherwise. It is moreover to be understood that, in case parameter ranges are given which are delimited by numeric values, the ranges are deemed to include these limitation values.

In one aspect, the present invention relates to a non-human mammal being partially or completely deficient in expressing a functional gene product encoded by the *Fto* gene.

In a preferred embodiment, the present invention relates to a non-human mammal being partially or completely deficient in expressing a functional gene product encoded by the *Fto* gene only.

According to the invention, it is to be understood that the non-human mammal according to the invention may be either transgenic, i.e. comprising at least one extrinsic nucleic acid sequence, or non-transgenic. In the latter case, the altered expression, particularly, the deprived expression, is due to a mutation in the *Fto* gene or its promoter which has been obtain with non transgenic methods, e.g. chemical or physical mutagenesis.

The "non-human mammal being partially or completely deficient in expressing a functional gene product encoded by the *Fto* gene" is also denoted in the present invention as "non-human knock-out mammal".

In another preferred embodiment, it is provided that the non-human knock-out mammal according to the invention is characterized in that said deficiency is due to a technique selected from the group comprising
a) insertion of a transposon into the *Fto* gene
b) site directed mutagenesis
c) gene targeting technique
d) application of chemical or physical mutagens, and subsequent selection of the respective genotype/phenotype
e) antisense RNA technique
f) RNA interference technique
g) modification or inactivation of a promoter operably linked to the *Fto* gene and/or
h) modification or inactivation of regulatory elements (enhancer/silencer) of the *Fto* gene.

Preferably, it is provided that the non-human knock-out mammal according to the invention is characterized in that said deficiency is due to a genetic alteration technique selected from the group comprising
a) insertion of a transposon into the *Fto* gene
b) site directed mutagenesis
c) gene targeting technique
d) application of chemical or physical mutagens, and subsequent selection of the respective genotype/phenotype
e) modification or inactivation of a promoter operably linked to the *Fto* gene and/or
f) modification or inactivation of regulatory elements (enhancer/silencer) of the *Fto* gene.

In a more preferred embodiment, it is provided that said genetic alteration technique comprises a gene targeting technique.

In a preferred embodiment, it is provided that the non-human knock-out mammal according to the invention is characterized in that said deficiency is due to genetic alteration based on a homozygous or heterozygous mutation in the *Fto* gene.

In a preferred embodiment, it is provided that the non-human knock-out mammal according to the invention is characterized in that said deficiency is due to genetic alteration based on a homozygous or heterozygous mutation in the *Fto* gene only. This is advantageous because no other genes are affected. It is thus possible to study the function of Fto without influence of disturbing genes.

Preferably, the mutation in the *Fto* gene according to the invention is selected from the group comprising an insertion, point mutation and/or deletion in the DNA sequence of the *Fto* gene, most preferred a deletion.

In a preferred embodiment, it is provided that the non-human knock-out mammal according to the invention is characterized in that said deficiency is due to genetic alteration based on a homozygous or heterozygous deletion in the *Fto* gene.

In a preferred embodiment, it is provided that the non-human knock-out mammal according to the invention is characterized in that said deficiency is due to genetic alteration based on a homozygous or heterozygous partial or total deletion in the *Fto* gene.

In a preferred embodiment, it is provided that the non-human knock-out mammal according to the invention is characterized in that said deficiency is due to genetic alteration based on a homozygous or heterozygous partial or total deletion in the *Fto* gene only.

In a preferred embodiment, it is provided that the non-human knock-out mammal according to the invention is characterized in that said deficiency is due to genetic alteration based on a partial deletion of the *Fto* gene only.

Preferably, said partial deletion is a heterozygous partial deletion. Preferably, said heterozygous partial deletion results in a leaky mutation of the endogenous gene encoding Fto. Said leaky mutation results in a partially disruption of the function of the Fto protein.

In another preferred embodiment, said partial deletion is a homozygous partial deletion. Preferably, said homozygous partial deletion results in a leaky mutation of the endogenous gene encoding Fto. Said leaky mutation results in a partially disruption of the function of the Fto protein. More preferably, said homozygous partial deletion results in a null mutation of the endogenous gene encoding Fto. Said null mutation results in a complete loss of Fto function.

Preferably, said partial deletion comprises the deletion of at least one *Fto* exon. Preferably, said partial deletion comprises the deletion of several Fto exons. More preferably, said partial deletion comprises the deletion of 2 to 3 *Fto* exons. In particular, said deletion comprises the deletion of exon 2 and exon 3 of the *Fto* coding sequence.

In another preferred embodiment, it is provided that the non-human knock-out mammal according to the invention is characterized in that said deficiency is due to genetic alteration based on a total deletion of the *Fto* gene only.

Preferably, said total deletion is a heterozygous total deletion. Preferably, said heterozygous total deletion results in a leaky mutation of the endogenous gene encoding Fto. Said leaky mutation results in a partially disruption of the function of the Fto protein.

More preferably, said total deletion is a homozygous total deletion. More preferably, said homozygous total deletion results in a null mutation of the endogenous gene encoding Fto. Said null mutation results in a complete loss of Fto function.

In a preferred embodiment, the deletion according to the invention comprises the insertion of a selectable marker sequence. Preferably, the selectable marker sequence is a neomycin resistance sequence. It is advantageous to insert a selectable marker sequence to confer resistance to a certain toxic agent for selection of non-human knock-out mammalian cells according to the invention.

Surprisingly, the inventors found that knock-out mice according to the invention seem to exhibit unchanged body proportions compared to wildtype mice. Another advantage is that said knock-out mice seem to exhibit unchanged anatomical characteristics. More preferably, said knock-out mice seem to exhibit no obvious deformities and no severe developmental defects. Furthermore, said knock-out mice seem to have a similar life-span than wildtype mice.

Furthermore, said knock-out mice are able to produce offspring. It is advantageous because said knock-out mice are fertile. Another advantage is that said knock-out mice are able to transmit the mutation according to the invention to said offspring.

The knock-out mammal according to the invention, preferably a knock-out mouse, can be generated by a process comprising the steps of: constructing a vector comprising a resistance cassette, preferably a neomycin resistance cassette, linearizing the vector, electroporating the linearized vector into the Embryonic Stem (ES) cells of an animal, preferably of a mouse, selecting the cells with the selection marker, incorporating the cells into an embryo, for example by microinjection into a blastocyst, that can be harvested by perfusing the uterus of pregnant females, reimplantating the embryo and selecting the transformed animals, followed by intercrossing to derive homozygous mutant F2 mammals, preferably mutant F2 mice.

The inventors of the present invention found that homozygous *Fto*^{*-*/*-*} knock-out mice display that their weight are clearly reduced. The inventors could demonstrate a weight reduction of homozygous knock-out mice of about 30 % and 40 %, respectively, compared to wildtype *Fto*^{+/+} mice, while heterozygous knock-out *Fto* ^{+/-} mice do not significantly differ from wildtype mice.

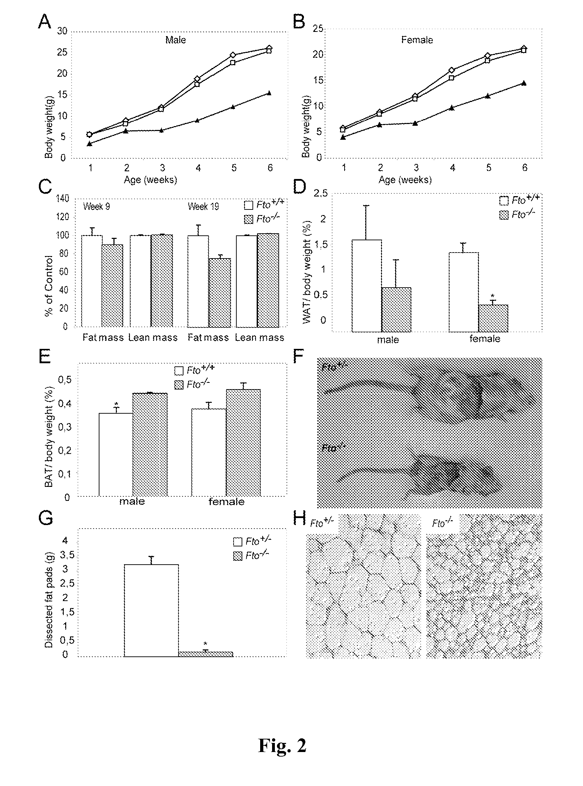

The inventors found that the lean phenotype of *Fto*^{*-*/*-*} mice presumably does not result from reduced caloric intake. Furthermore, the inventors could demonstrate that *Fto*^{*-*/*-*} mice show a clear reduction of white adipose tissue (WAT) in correlation to body weight while the content of brown adipose tissue (BAT) was increased. A higher content of BAT may indicate increased energy expenditure in *Fto*^{*-*/*-*} mice (see Fig. 2 D, E).

The inventors could demonstrate that leanness of Fto-deficient mice may develop as a consequence of increased energy expenditure possibly due to an increased BAT mass.

Thus, the inventors demonstrate for the first time that the knock-out mouse according to the invention is a useful system for studying the physiological role of Fto.

Additionally, the inventors found that when the knock-out mice are fet with fat diet instead of standard diet the weight gain of the heterozygous *Fto*^{+/+} knock-out mice is significantly reduced compared to the weight gain of the wildtype *Fto*^{+/+} mice. Thus, under these conditions even heterozygous *Fto*^{+/-} knock-out mice differ significantly from wildtype *Fto*^{+/+} mice. This shows that even a heterozygous *Fto*^{+/-} knock-out mouse is a useful system for studying the physiological role of Fto.

Furthermore, the inventors found that homozygous *Fto*^{-/-} knock-out mice display a postnatal growth retardation and found that their size is clearly reduced. Without being bound to any theory, it is assumed that Fto is involved in processes connected to growth regulation.

This shows that the mouse according to the invention is a useful system for studying mechanisms of disturbances of growth, preferably of dwarfism.

In another aspect, the present invention relates to a non-human mammal, said mammal being characterized in that the expression level of an Fto protein is increased.

The "non-human mammal being characterized in that the expression level of an Fto protein is increased" is also denoted in the present invention as "Fto-overexpressing non-human mammal".

In a preferred embodiment, it is provided that the Fto-overexpressing non-human mammal according to the invention is characterized in that the genome of said mammal comprises a transgenic nucleotide sequence encoding for the Fto protein, and wherein said nucleotide sequence is operably linked to a promoter.

In preferred embodiments, the transgenic nucleotide sequence for generating an Fto-overexpressing non-human mammal according to the invention is an *Fto* cDNA sequence comprising the sequence of SEQ ID NO: 1. Preferably, the *Fto* gene encodes a protein comprising the amino acid sequence of SEQ ID NO: 2. The GenBank accession number for the related transcript is NM_011936.

Preferably, expression of the *Fto* gene can be increased only conditionally, using promoters that are either site- or time-specific, or inducible promoters. It is thus possible to increase *Fto* expression only in specific tissues.

In a preferred embodiment, it is provided that the promoter according to the invention exerts its activity in a tissue selected from the group comprising liver, connective tissue, muscle, kidneys, lungs, spleen, pancreas, in particular in the islets of Langerhans, and/or salivary glands. In a more preferred embodiment, it is provided that the promoter according to the invention exerts its activity in a tissue selected from the group comprising liver, connective tissue, muscle and/or pancreas, in particular in the islets of Langerhans.

In another preferred embodiment, it is provided that said promoter comprises murine major histocompatibility complex promoter H2-K, said promoter comprises the nucleic acid sequence according to SEQ ID NO: 3 (Rüther U et al., Cell 1988; 53(6): 847-56). It is advantageous to use said promoter because the transgene is stable and highly expressed.

The insertion of a construct in the genome of an animal to obtain an Fto-overexpressing animal, with an Fto-overexpressing mouse as an example of the Fto-overexpressing animal, may be performed by methods well known by the person skilled in the art, and can be either random or targeted. In a few words, the person skilled in the art will construct a vector comprising the sequence to insert within the genome, with an appropriate promoter. Said vector may be microinjection into the zygote of an animal, preferably of a mouse. The zygote is isolated by perfusing the oviduct of pregnant females. Reimplantation of the embryo and selection of the transformed animals, preferably of transformed mice, followed by potential back-crossing makes it possible to obtain such Fto-overexpressing animal, preferably Fto-overexpressing mouse.

In another preferred embodiment of the present invention, it is provided that upstream of the transgenic nucleotide sequence for generating an Fto-overexpressing non-human mammal according to the invention an intron is introduced.

Preferably, the introduced intron is the second intron of the β-globin gene from rabbit.

It is advantageous to introduce said intron into an *Fto* cDNA construct because this increases the expression levels.

In a preferred embodiment, it is provided that the Fto-overexpressing non-human mammal according to the invention comprises a transgene construct having the H2-K promoter sequence, the second intron of the β-globin gene from rabbit and the *Fto* cDNA, wherein said construct comprises
a) a nucleic acid molecule having the nucleotide sequence of SEQ ID NO: 4, or a fragment, variant, homologue, or derivative thereof, and/or
b) a nucleic acid molecule having a sequence identity of at least 70, preferably 95 % with any of the nucleic acid molecules of a).

Preferably, the expression products of *Fto* according to the invention comprise RNA and/or protein.

Preferably, the expression of the *Fto* gene is a stable expression.

In a preferred embodiment of the present invention, it is provided that the non-human mammal according to the invention is a rodent.

In a more preferred embodiment of the present invention, it is provided that the non-human mammal according to the invention is a mouse or a rat. In a more preferred embodiment, it is provided that the non-human mammal according to the invention is a mouse. Said mouse according to the invention is a useful system for studying the physiological role of Fto.

In a most preferred embodiment, it is provided that the non-human mammal according to the invention is a mouse derived from a C57/BL6 x C3H mating. Said mouse derived from a C57/BL6 x C3H mating according to the invention is a particularly suitable biological system for studying the physiological role of Fto.

In one aspect of the present invention, a cell, cultured cell, cell line, cell culture and/or tissue culture derived from a non-human mammal according to the present invention is provided.

In one aspect of the present invention, use of a non-human mammal according to the present invention, and/or a cell, cultured cell, cell line, cell culture and/or tissue culture according to the present invention is provided as a model for the study of diseases correlated with the expression of *FTO* in a patient.

In a preferred embodiment of the present invention, it is provided that the patient is human.

In a preferred embodiment, said disease correlated with the expression of *FTO* in a patient is selected from the group comprising obesity, type II diabetes, a cardiovascular disease, and/or dwarfism. Preferably, it is provided that said cardiovascular diseases are selected from the group comprising stroke, atherosclerosis, cardiomyopathy, myocarditis and/or high blood pressure. Type II diabetes and/or cardiovascular diseases may develop as a consequence of obesity.

In another preferred embodiment, the present invention relates to cells, cell cultures and/or cell lines derived from mice of different genotypes, that is *Fto* ^{+/+}, *Fto* ^{+/-} and/or *Fto* ^{-/-}. The inventors could demonstrate that embryonic fibroblasts derived from Fto-deficient mice according to the present invention can proliferate and that passaging of said embryonic fibroblasts is possible.

One advantage of the use of a non-human mammal according to the present invention and/or a cell, cultured cell, cell line, cell culture and/or tissue culture according to the present invention is that it allows to analyse which other genes than *Fto* exhibit a differently regulated, that is upregulated or downregulated, expression level in a non-human knock-out mammal and/or in an Fto-overexpressing non-human mammal.

Preferably, a microarray analysis may be used to identify differently regulated, that is upregulated or downregulated, genes in a non-human mammal according to the present invention and/or in a cell, cultured cell, cell line, cell culture and/or tissue culture derived thereof according to the present invention.

In one aspect of the present invention, use of a non-human mammal according to the present invention, and/or of a cell, cultured cell, cell line, cell culture and/or tissue culture according to the present invention is provided for screening of a compound having therapeutic activity comprising
a) administering a test compound to the non-human mammal or to the cell, cultured cell, cell line, cell culture and/or tissue culture, and
b) evaluating the effect of said compound on said non-human mammal according or cell, cultured cell, cell line, cell culture and/or tissue culture.

In a particularly preferred embodiment, the non-human mammal used in this embodiment is an Fto-overexpressing non-human mammal.

However, in one embodiment, the non-human mammal used in this embodiment is a non-human knock-out mammal according to the invention. Said non-human knock-out mammal may be used as a model to evaluate the molecular consequences of a treatment directed against Fto on a non-human mammal. This knowledge may be used as a suitable approach for the selection of compounds of interest. Preferably, said evaluation of the molecular consequences is performed by microarray analyses.

In a preferred embodiment of the invention, it is provided that evaluating the effect of said compound on said Fto-overexpressing non-human mammal according to the present invention is the evaluation whether or not the expression level of *Fto* is reduced in the Fto-overexpressing non-human mammal and/or the cell, cultured cell, cell line, cell culture and/or tissue culture derived thereof according to the present invention.

In a more preferred embodiment of the invention, it is provided that evaluating the effect of said compound on said Fto-overexpressing non-human mammal according to the present invention is the evaluation whether or not obesity is reduced in the Fto-overexpressing non-human mammal. In particular, the weight of the Fto-overexpressing non-human mammal according to the invention is evaluated.

In another preferred embodiment, it is provided that evaluating the effect of said compound on said Fto-overexpressing non-human mammal according to the invention is the evaluation whether or not an obesity associated disease is reduced in the Fto-overexpressing non-human mammal. Preferably, it is provided that said obesity associated disease is selected from the group comprising type II diabetes and/or cardiovascular diseases, such as stroke, atherosclerosis, cardiomyopathy, myocarditis and high blood pressure.

In another preferred embodiment, it is provided that evaluating the effect of said compound on said Fto-overexpressing non-human mammal according to the invention is the evaluation whether or not growth is reduced in the Fto-overexpressing non-human mammal. In particular, the size of the Fto-overexpressing non-human mammal according to the invention is evaluated.

In a preferred embodiment of the present invention, it is provided that the compound is selected from the group comprising an antibody, a protease, a small molecule drug (SMD) and/or an antisense construct.

Compounds of interest are compounds directed against FTO as well as compounds directed against interaction partners of FTO. Methods for identifying interaction partners, for instance kinases, preferably are selected via the yeast two-hybrid system and/or the GST pulldown assay.

In one aspect, the present invention relates to an antibody, a protease and/or a small molecule drug directed against an FTO protein of human origin and/or an interaction partner thereof and/or directed against an Fto protein of non-human origin and/or an interaction partner thereof.

Preferably, the *FTO* cDNA sequence comprises the sequence of SEQ ID NO: 9. Preferably, the *FTO* gene encodes a protein comprising the amino acid sequence of SEQ ID NO: 10. The GenBank accession number for the related transcript is NM_001080432.

In a preferred embodiment of the present invention, it is provided that the antibody according to the invention is directed against an FTO protein and/or against an interaction partner of said FTO protein, wherein said FTO protein
a) is encoded by the nucleic acid of SEQ ID NO: 9, or a fragment, variant, homologue or derivative thereof,
b) is encoded by a nucleic acid molecule that is capable of hybridizing to any of the nucleic acid molecules of a) under stringent conditions,
c) is encoded by a nucleic acid molecule having a sequence identity of at least 70, preferably 95 % with any of the nucleic acid molecules of a) or b),
d) is encoded by a nucleic acid molecule that is capable of hybridizing to the complement of any of the nucleic acid molecules of a) - c) under stringent conditions,
e) is encoded by a nucleic acid molecule which comprises, in comparison to the nucleic acid molecule according to a) - d) at least one silent single nucleotide mutation (as allowed by the degeneracy of the genetic code),
f) is encoded by a nucleic acid molecule which, in comparison to the nucleic acid molecule according to a) - e), is code optimized for a given expression host,
g) comprises an amino acid sequence according to SEQ ID NO: 10, or a fragment, variant, homologue or derivative thereof, and/or
h) comprises an amino acid sequence having a sequence identity of at least 70, preferably 95 % with any of the amino acid sequences of g).

Methods for generating antibodies according to the invention are well known in the state of the art (Delves PJ "Antibody production: essential techniques", New York, John Wiley & Sons 1997; pp. 90-113). For generating an antibody against a given molecule (the antigen), a strategy is to immunize the animal of choice with a highly purified preparation of the molecule. The immunization will be done repeatedly at 2-3 week intervals to get the levels of antibody as high as possible. After repeated immunizations, the animals will be bled and the sera will be tested for reactivity against the injected antigen.

The antibody according to the invention may be monoclonal, polyclonal, recombinant, chimeric, single-chain and/or bispecific. In a preferred embodiment, the antibody or fragment thereof will either be of human origin, or will be "humanized", i.e., prepared so as to prevent or minimize an immune reaction to the antibody when administered to a patient.

Antibodies directed against an FTO protein and/or against an interaction partner of said FTO protein may be used to reduce the FTO expression level in a patient. Said antibodies may be used for the treatment of a disease correlated with the expression of *FTO,* preferably obesity, type II diabetes, a cardiovascular disease, and/or dwarfism.

In a preferred embodiment of the present invention, it is provided that the protease according to the invention is directed against an FTO protein and/or against an interaction partner of said FTO protein, wherein said FTO protein
a) is encoded by the nucleic acid of SEQ ID NO: 9, or a fragment, variant, homologue or derivative thereof,
b) is encoded by a nucleic acid molecule that is capable of hybridizing to any of the nucleic acid molecules of a) under stringent conditions,
c) is encoded by a nucleic acid molecule having a sequence identity of at least 70, preferably 95 % with any of the nucleic acid molecules of a) or b),
d) is encoded by a nucleic acid molecule that is capable of hybridizing to the complement of any of the nucleic acid molecules of a) - c) under stringent conditions,
e) is encoded by a nucleic acid molecule which comprises, in comparison to the nucleic acid molecule according to a) - d) at least one silent single nucleotide mutation (as allowed by the degeneracy of the genetic code),
f) is encoded by a nucleic acid molecule which, in comparison to the nucleic acid molecule according to a) - e), is code optimized for a given expression host,
g) comprises an amino acid sequence according to SEQ ID NO: 10, or a fragment, variant, homologue or derivative thereof, and/or
h) comprises an amino acid sequence having a sequence identity of at least 70, preferably 95 % with any of the amino acid sequences of g).

Proteases according to the invention may be generated by protein engineering approaches well known in the state of the art, preferably by a directed evolution approach (see definitions).

It is advantageous to use proteases because proteases are in general smaller molecules than antibodies. Another advantage is that proteases show less immunogenicity in a patient. Furthermore, proteases directed against an FTO protein and/or against an interaction partner of said FTO protein are very specific towards their target molecules.

Proteases directed against an FTO protein and/or against an interaction partner of said FTO protein may be used to reduce the FTO expression level in a patient. Said proteases may be used for the treatment of a disease correlated with the expression of *FTO,* preferably obesity, type II diabetes, a cardiovascular disease, and/or dwarfism.

In a preferred embodiment of the present invention, it is provided that the small molecule drug according to the invention is directed against an FTO protein and/or against an interaction partner of said FTO protein, wherein said FTO protein
a) is encoded by the nucleic acid of SEQ ID NO: 9, or a fragment, variant, homologue or derivative thereof,
b) is encoded by a nucleic acid molecule that is capable of hybridizing to any of the nucleic acid molecules of a) under stringent conditions,
c) is encoded by a nucleic acid molecule having a sequence identity of at least 70, preferably 95 % with any of the nucleic acid molecules of a) or b),
d) is encoded by a nucleic acid molecule that is capable of hybridizing to the complement of any of the nucleic acid molecules of a) - c) under stringent conditions,
e) is encoded by a nucleic acid molecule which comprises, in comparison to the nucleic acid molecule according to a) - d) at least one silent single nucleotide mutation (as allowed by the degeneracy of the genetic code),
f) is encoded by a nucleic acid molecule which, in comparison to the nucleic acid molecule according to a) - e), is code optimized for a given expression host,
g) comprises an amino acid sequence according to SEQ ID NO: 10, or a fragment, variant, homologue or derivative thereof, and/or
h) comprises an amino acid sequence having a sequence identity of at least 70, preferably 95 % with any of the amino acid sequences of g).

For generating a small molecule drug directed against an FTO protein and/or against an interaction partner of said FTO protein, the three-dimensional structure of said FTO protein and/or of said interaction partner of the FTO protein has to be determined. Small molecule drugs can target for instance enzyme- or receptor-binding sites to inhibit their target proteins.

It is advantageous to use small molecule drugs because they are in general smaller molecules than antibodies. Another advantage is that small molecule drugs show less immunogenicity in a patient. Furthermore, small molecule drugs directed against an FTO protein and/or against an interaction partner of said FTO protein are very specific towards their target molecules.

Small molecule drugs directed against an FTO protein and/or against an interaction partner of said FTO protein may be used to reduce the FTO expression level in a patient. Said small molecule drugs may be used for the treatment of a disease correlated with the expression of FTO, preferably obesity, type II diabetes, a cardiovascular disease, and/or dwarfism.

In one aspect of the present invention, an antisense construct directed against a nucleic acid related to the *Fto* gene of non-human origin and/or directed against a nucleic acid related to the *FTO* gene of human origin is provided.

In a preferred embodiment, the antisense construct according to the invention directed against a nucleic acid related to the *FTO* gene is provided, wherein said *FTO* gene comprises a nucleic acid sequence of SEQ ID NO: 9, or a fragment, variant, homologue or derivative thereof.

In order to decrease the activity of the *FTO* gene and protein, it is possible to use antisense constructs, in particular complementary to the mRNA of *FTO.* The person skilled in the art is aware of the means to design antisense constructs, and of the modifications that can be brought to the backbones of the molecules, for example phosphorothioates and/or methylphosphonates.

Said antisense constructs may be used for the treatment of a disease correlated with the expression of *FTO,* preferably obesity, type II diabetes, a cardiovascular disease, and/or dwarfism.

In a preferred embodiment, it is provided that the antisense construct according to the present invention is characterized in that this construct comprises a nucleotide sequence complementary to
a) the nucleic acid of SEQ ID NO: 9, or a fragment, variant, homologue or derivative thereof ,
b) a nucleic acid molecule having a sequence identity of at least 70, preferably 95 % with any of the nucleic acid molecules of a),
c) a nucleic acid molecule which comprises, in comparison to the nucleic acid molecule according to a) - b) at least one silent single nucleotide mutation (as allowed by the degeneracy of the genetic code), and/or
d) a nucleic acid molecule which, in comparison to the nucleic acid molecule according to a) - c), is code optimized for a given expression host.

In one aspect of the present invention, the antibody, the protease, the SMD and/or the antisense construct according to the present invention is provided for use as pharmaceutic and/or a diagnostic composition.

In another aspect of the present invention, the antibody, the protease, the SMD and/or the antisense construct according to the present invention is provided for the treatment, prevention and/or diagnosis of a disease correlated with the expression of *FTO,* preferably obesity, type II diabetes, a cardiovascular disease, and/or dwarfism.

In yet another embodiment of the invention, it is provided to use the antibody, the protease, the SMD and/or the antisense construct according to the present invention in the manufacture of a pharmaceutic for the treatment, prevention and/or diagnosis of a disease correlated with the expression of *FTO,* preferably obesity, type II diabetes, a cardiovascular disease, and/or dwarfism.

Said cardiovascular diseases comprise stroke, atherosclerosis, cardiomyopathy, myocarditis and/or high blood pressure.

### Brief description of the examples and drawings

Additional details, features, characteristics and advantages of the object of the invention are disclosed in the subclaims, and the following description of the respective figures and examples, which, in an exemplary fashion, show preferred embodiments of the present invention. However, these drawings should by no means be understood as to limit the scope of the invention.

### Figures

Figure 1A demonstrates a Western-Blot analysis of extracts from *Fto*^{+/+} (1) and *Fto*^{-/-} (2) mouse embryonic fibroblasts.
Figure 1B demonstrates an Immunofluorescence analysis in *Fto*^{+/+} and *Fto*^{-/-} cells with the Fto-specific antibody.
Figures 2A - H demonstrate the phenotypic characteristics of Fto-negative mice.
Figures 3 A - C demonstrate the energy metabolism of *Fto*^{-/-} mice.
Figures 4 A and B demonstrate the weight gain of *Fto*^{+/+} and *Fto*^{+/*-*} mice (males and females) after feeding with high fat diet.

### Materials and Methods

### Animal care

Mice were housed in a virus-free facility at 22-24°C on a 12 h light/ 12 h dark cycle with the light on at 6 a.m. and were fed standard rodent chow (Ssniff, Soest, Germany). All animals had access to water *ad libitum.* All animal procedures and euthanasia were reviewed by the animal care committee of the respective institution, approved by local government authorities and were in accordance with National Institutes of Health guidelines. Body weight was determined once a week. Food intake was measured over a period of 7 days and daily food intake was calculated as the average intake of chow within the time stated.

### Immunofluorescence

Cells were grown on cover slips until 80% confluency. They were washed with ice cold PBS (Biochrom AG) and fixed with 4%PFA. After three washing steps with PBS, cells were permeabilized with PBS with 0.5%Triton X 100. Blocking was performed with 10% FCS in PBS with 0.1% TritonX100 (FCS/PBT). The cells were incubated with the antibodies diluted in FCS/PBT for 2h (primary) and 30min (secondary) with three washing steps after the incubation with the primary antibodies. The cells were then washed again and embedded in Mowiol containing DAPI (Merck).

### Analysis of Body Composition

Nuclear magnetic resonance (NMR) was used to determine whole body composition of live animals using the NMR Analyzer minispec mq7.5 (Bruker Optik, Ettlingen, Germany).

### Hematoxylin and eosin (HE) staining

After dissection in PBS, tissues were fixed in 4% paraformaldehyde over night and washed in PBS. They were incubated in 70, 80, 90, 100% ethanol for 2h, respectively, in 1-butanol over night and then transferred into paraffin for embedding. Staining on paraffin sections was performed with hematoxylin and eosin.

### Indirect calorimetry

Indirect calorimetry was measured in a Calorimetry Module (CaloSys V2.1, TSE Systems, Bad Homburg, Germany). After two hours of acclimatization, parameters of indirect calorimetry were measured for at least 24 hours.

### Statistical Methods

Unless stated otherwise, all values are mean ± SEM. Data sets were analyzed for statistical significance using a two-tailed unpaired student's t test. All p values below 0.05 were considered significant.

### Antibodies

Mouse antibody against acetylated α-tubulin was from Sigma and secondary antibodies conjugated to HRP and Cy2 and Cy3 were from DAKO and Dianova, respectively.

### SDS gelelectrophoresis and Western Blotting

For SDS electrophoresis cells were lysed in sample buffer and analyzed by Western blotting. Cells were washed in ice cold PBS and lysed in SDS sample buffer (prepared as 4x stock: 8ml 1M Tris pH 6.8, 2g SDS stock, 10ml glycerol, 0.02% bromophenol blue, 6ml water, 20% β-mercaptoethanol) (Laemmli UK, Nature 1970; 227: 680-685). After SDS gelelectrophoresis proteins were transferred onto a PVDF membrane by Western Blotting (Kyshe L and Anderson J; J. Biochem. Biophys. Methods. 1984; 10: 203-209). The membrane was blocked in blocking solution (5% milk powder in PBS with 0.1% Tween). Antibodies were diluted in blocking solution and incubation was performed at room temperature for 1h. After antibody incubation the membrane was washed several times with PBS with 0.1% Tween). Signals were detected with ECL Western Blotting Detection Reagents (GE Healthcare).

### Example 1

### Generation of an Fto-knock-out mouse

For the targeted deletion of *Fto,* the inventors designed a construct to introduce a neomycin resistance cassette resulting in a deletion of exon 2 and exon 3 of the *Fto* coding sequence. The replaced region spans the sequence from SpeI restriction site position 93924726 in Mus musculus Chr.8 (Mm8), which is a mouse genome database produced by NCBI to BamHI restriction site position 93934002 in Mm8. The linearized vector was electroporated into R1 mouse embryonic stem (ES) cells (Nagy A at al., Proc. Natl. Acad. Sci. USA 1993; 90: 8424-8428); positive clones were selected by G418 (Gibco BRL) application, which is an aminoglycoside antibiotic, and tested by Southern Blot analysis. After separation via gel electrophoresis the DNA was transferred onto a nylon membrane using alkaline transfer (Southern, J. Mol. Biol. 1975; 98: 503ff.). The membrane was then baked for 1h, 80°C, prepared for hybridization in 1M NaCl, 50mM Tris, pH 8, 1% SDS. Hybrisization was performed with a radiolabeld DNA probe in 1M NaCl, 50mM Tris, pH 8, 1% SDS, 10% dextrasulfate. The membrane was washed in Saline Sodium Citrate (SSC) several times at 65°C. For detection the membrane was exposed to an X-ray film for autoradiography. Targeted ES cells were used to create chimeras that transmit the *Fto* mutation to their progeny. F1 animals were intercrossed to derive homozygous mutant F2 mice.

Genotyping was carried out by PCR analysis of DNA extracted from tails or from the yolk sac of embryos (Saiki R et al., Science 1985; 230:1350-1354). The following primers were used: Fto KO for: acccctctcccccatctaaatcct according to SEQ ID NO: 5, Fto KO rev: aagccaagaacaagtccatacctg according to SEQ ID NO: 6, Neo for: ctgtgctcgacgttgtcactg according to SEQ ID NO: 7, Neo rev: gatcccctcagaagaactcgt according to SEQ ID NO: 8. PCR was performed using the following program: 5min 95°C; 35 cycles: 1min 95°C, 1min 56°C, 1min 72°C; 5min 72°C.

Genotyping showed that the inventors could generate Fto-knock-out mice, that is *Fto*^{-/-} mice and *Fto*^{+/-} mice.

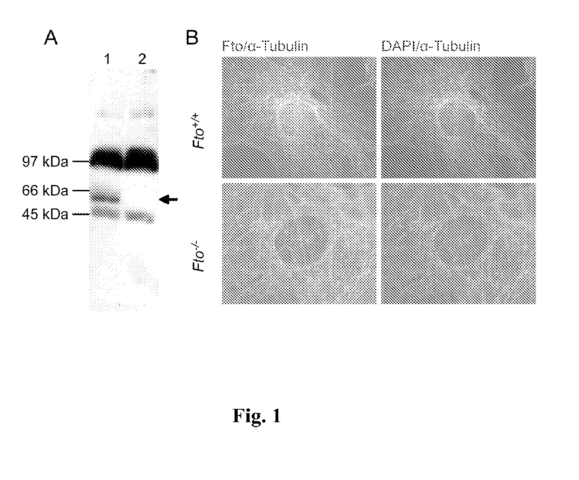

Successful deletion of *Fto* was confirmed as well by immunoblotting and immunofluorescence analysis (Fig. 1A, B) of mouse embryonic fibroblasts derived from littermates of *Fto*^{+/-} x *Fto*^{+/-} matings of embryos of the E12.5 stage. Counterstaining was performed with α-tubulin antibody and DAPI.

The Fto protein was detected in cells derived from *Fto*^{+/+} but not in cells from *Fto*^{-/-} animals. These analyses also confirm the calculated molecular mass of ∼58 kDa (Fig. 1 A) and show the nuclear localization of the endogenous protein (Fig. 1 B).

Thus, immunoblotting and immunofluorescence analysis showed as well that the inventors could generate Fto-knock-out mice.

*Fto*^{-/-} mice were born at the expected Mendelian ratio suggesting that Fto is not essential for embryonic development.

The inventors of the present invention could generate for the first time Fto-deficient *Fto*^{-/-} mice.

### Example 2

### Studies of the effects of the Fto-deficiency

The inventors monitored the body weight over a period of six weeks in 1 to 6 weeks old mice and detected an average weight reduction of 6.7g in females and of 10.6g in males after 6 weeks. This corresponds to a weight reduction of homozygous mice of about 30% and 40%, respectively, compared to control littermates, while *Fto*^{+/-} mice do not significantly differ from *Fto*^{+/+} in either gender (Fig. 2A, B; 0 *Fto*^{+/+}, □ *Fto*^{+/-}, ▲ *Fto*^{-/-}, female: *Fto*^{+/+}: =9, *Fto*^{+/-}: n=18, *Fto*^{-/-}: n=3; male: *Fto*^{+/+}: n=7, *Fto*^{+/-}: n=12, *Fto*^{-/-}: n=3).

The inventors could demonstrate that the effects of the Fto deficiency manifest shortly after birth as *Fto*^{-/-} mice display a postnatal growth retardation, their size and their weight are clearly reduced. The body proportions and the anatomical characteristics appear unchanged. The smaller size and the leanness of the *Fto*^{-/-} mice remain obvious throughout their whole lifespan.

The inventors could demonstrate that an Fto-deficient mouse is a useful system for studying mechanisms of obesity and/or disturbances of growth, preferably of dwarfism.

### Example 3

### Studies of the effects of the Fto-deficiency on the fat content

Considering the suggested function of Fto in obesity development, the inventors examined the fat content of the animals by magnetic resonance imaging (MRI).

At the age of nine weeks, the body composition of *Fto*^{-/-} mice does not significantly differ from *Fto*^{+/+} mice (Fig. 2 C). After 19 weeks a tendency towards reduced body fat mass can be seen in *Fto*^{-/-} mice, but this does not reach statistical significance. For further analysis, the inventors dissected the 20 weeks old animals and determined the weight of isolated brown and white adipose tissue (BAT, WAT).

The inventors observed a clear reduction of WAT in correlation to body weight (Fig. 2 D) while the content of BAT was increased in *Fto*^{-/-} mice (Fig. 2 E). A higher content of BAT therefore may indicate increased energy expenditure in *Fto*^{-/-} mice.

They could demonstrate that leanness of Fto-deficient mice may develop as a consequence of increased energy expenditure possibly due to an increased BAT mass.

The inventors could demonstrate that an Fto-deficient mouse is a useful system for studying mechanisms of obesity.

### Example 4

### Studies of the effects of the Fto-deficiency on the adipose tissue

As the fat mass of mice increases with age, the inventors analyzed 12 to 15 months old Fto-deficient mice (n=3). When the inventors dissected *Fto*^{+/-} mice as control (n=3), large inguinal and abdominal fat pads were clearly present. In contrast, *Fto*^{-/-} mice show hardly any adipose tissue mass at all (Fig. 2 F). The inventors isolated the inguinal fat and the adipose tissue from the body cavity for weight comparison. The fat mass of *Fto*^{-/-} mice was reduced by about 95% compared to that of control animals (Fig. 2 G; n=3).

Further, the inventors examined the adipose tissue histologically. Hematoxylin and eosin (HE) staining showed an overall reduced and heterogeneous adipocyte size in *Fto*^{-/-} mice (Fig. 2 H). This phenomenon is often associated with phenotypes protected against obesity.

This shows that Fto-deficiency leads to a reduced adipose tissue mass. The inventors could demonstrate that an Fto-deficient mouse is a useful system for studying mechanisms of obesity.

### Example 5

### Studies of the effects of the Fto-deficiency on the food intake

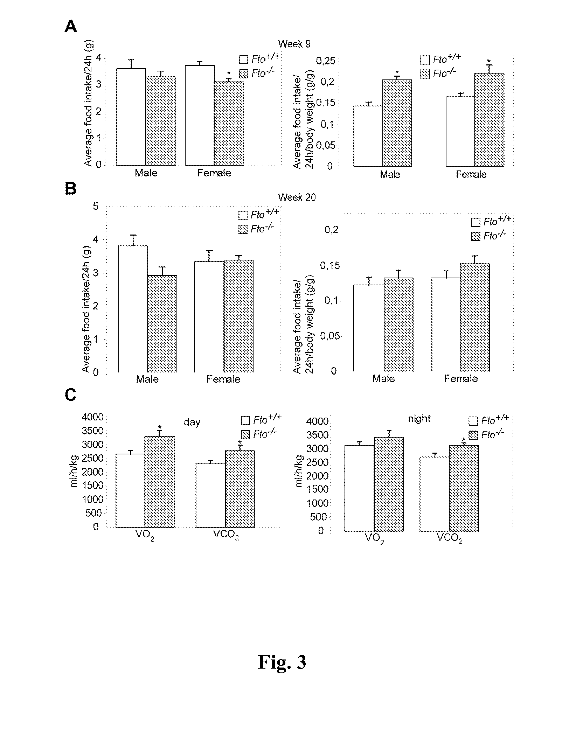

Daily food intake was measured over the course of 7 days. In total, the food intake of nine weeks old *Fto*^{-/-} mice (Fig. 3 A; *Fto*^{+/+}: n=6, *Fto*^{-/-}: n=6) and 20 weeks old *Fto*^{-/-} mice (Fig. 3 B; *Fto*^{+/+}: n=5, *Fto*^{-/-}: n=3), respectively, was decreased in both genders compared to littermate controls. After correcting the data for body weight (relative food intake), however, homozygous *Fto*^{-/-} mice showed an increased food intake compared to wildtype *Fto*^{+/+} mice. The inventors detected an average increased food intake of 42% in 9 weeks old males, of 38% in 9 weeks old females, of 8% in 20 weeks old males and of 15% in 20 weeks old females (Fig. 3A, B).

These experiments indicate that the lean phenotype of *Fto*^{-/-} mice presumably does not result from reduced caloric intake.

Therefore, the inventors measured energy expenditure of the animals through a Comprehensive Lab Monitoring System (CLAMS). The oxygen (O₂) consumption and carbon dioxide (CO₂) production of *Fto*^{-/-} mice were significantly increased during the day period compared to control animals (O₂: 27%, CO₂: 23%) and CO₂ production remained significantly increased during night time (O₂: 14%, CO₂: 19%), indicating a higher energy expenditure of *Fto*^{-/-} mice in comparison with their *Fto*^{+/+} littermates (Fig. 3 C; *Fto*^{+/+}: n=4, *Fto*^{-/-}: n=3).

The inventors could demonstrate that leanness of Fto-deficient mice may develop as a consequence of increased energy expenditure possibly due to an increased BAT mass.

The inventors could demonstrate that an Fto-deficient mouse is a useful system for studying mechanisms of obesity.

### Example 6

### Studies of the effects of high fat diet on Fto-knock-out mice

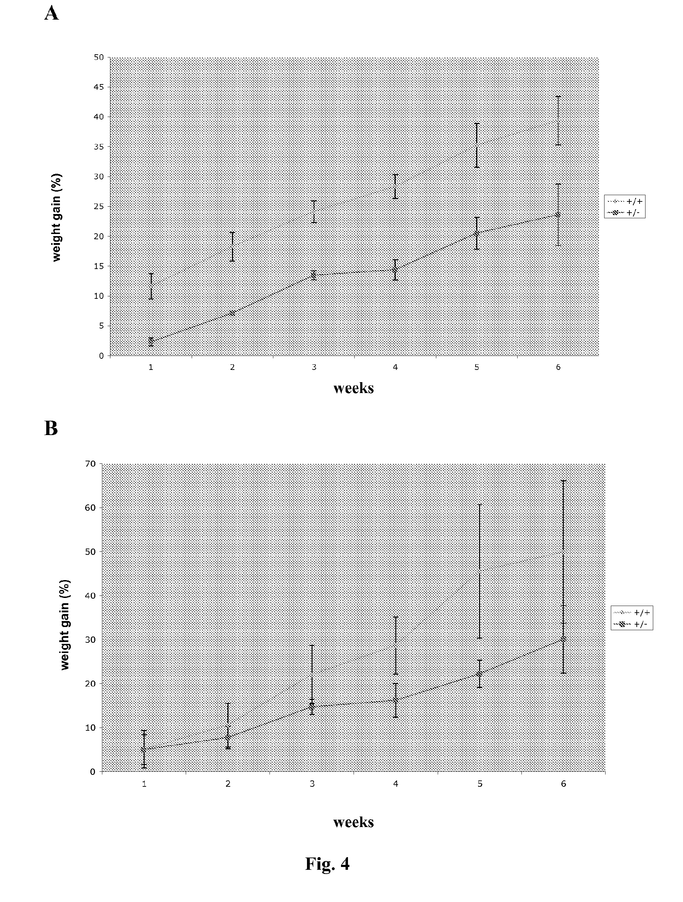

The inventors of the present invention fed 7 to 10 weeks old *Fto*^{+/+} (n=6, males and n=4, females) and *Fto*^{+/-} (n=5, males and n=6, females) mice over a period of 6 weeks with high fat diet (Ssniff, Soest, Germany) to test whether under these diet conditions, a difference in weight gain between *Fto*^{+/+} and *Fto*^{+/-} mice can be observed. The mice had access to the food *ad libitum.* Surprisingly, it could be demonstrated that the weight gain of *Fto*^{+/-} mice is strongly reduced (males: reduced by 38%, females: reduced by 40%) compared to *Fto*^{+/+} mice in either gender (Fig. 4A, B).

Thus, under these conditions it is not required to use *Fto*^{-/-} mice to observe a negative influence on obesity development.

The inventors of the present invention show for the first time the consequences of Fto-deficiency in Fto-knock-out mice, that is the reduction of their weight. They could demonstrate for the first time that the knock-out mouse according to the invention is a useful system for studying mechanisms of obesity, and that even a heterozygous *Fto*^{+/-} mouse is a useful system for this study.

### Example 7

### Generation of an Fto polyclonal antibody

A polyclonal antibody was raised in rabbits immunized with recombinant Fto according to standard procedures (Pineda antibody services). The antibodies were affinity-purified with the antigen coupled to NHS-activated sepharose 4 fast flow (GE Healthcare).

The construct for the production of an antibody (pASK-IBA45-Fto) comprises the complete *Fto* cDNA (SEQ ID NO: 1), N-terminal the sequence encoding a Strep-tag and C-terminal the sequence encoding a His-tag.

### Example 8

### Generation of mouse embryonic fibroblasts from knock-out mice

Mouse embryonic fibroblasts were derived from littermates of *Fto*^{+/-} x *Fto*^{+/-} matings of embryos of the E12.5 stage. They were maintained in Dulbecco's modified Eagle's Medium (Gibco BRL) supplemented with 10% fetal calf serum (PAA), 1% (v/v) L-Glutamine (Gibco, BRL) and 1% (v/v) PenStrep (Gibco, BRL) at 37°C in a humidified CO₂ incubator.

Successful deletion of *Fto* was confirmed by immunoblotting and immunofluorescence analysis (Fig. 1A, B). Counterstaining was performed with α-tubulin antibody and DAPI.

The Fto protein was detected in cells derived from *Fto*^{+/+} but not in cells from *Fto*^{-/-} animals. These analyses confirm the calculated molecular mass of ∼58 kDa (Fig. 1 A) and show the nuclear localization of the endogenous protein (Fig. 1 B).

The cells were cultured for 4 weeks (10 passages). The inventors could demonstrate that embryonic fibroblasts derived from Fto-deficient mice according to the present invention can proliferate and that passaging of said embryonic fibroblasts is possible.

### Example 9

### Generation of an Fto-overexpressing mouse

The procedure for generating an Fto-overexpressing mouse was performed in accordance with the method described in Murphy C et al., Microinjection and Transgenesis. Strategies and protocols. 1998; eds. A. Cid-Arregui & A. Garcia-Carranca. Springer Heidelberg, Springer Laboratory Manual, pp. 275-284, unless otherwise noted.

The used transgene construct comprising the nucleotide sequence of SEQ ID NO: 4 comprises the murine major histocompatibility complex promoter H2-K comprising the nucleotide sequence of SEQ ID NO: 3, the second intron of the β-globin gene from rabbit and the *Fto* cDNA comprising the nucleotide sequence of SEQ ID NO: 1. For the construction of said transgene construct a 0.640 kb BamHI, EcoRI fragment carrying the second intron of the β-globin gene from rabbit was cloned into the BamHI, EcoRI site of plasmid pUC H2 (Morello D et al., EMBO J 1986; 5: 1877-1883). Likewise, a 2.388 kb EcoRI fragment carrying the *Fto* cDNA comprising the nucleotide sequence of SEQ ID NO: 1 was cloned into the EcoRI site of said plasmid. This plasmid contains a 2 kb H2-K promoter fragment including the first 9 bases of the H2 mRNA. For microinjection, a 6.4kb HindIII, KpnI DNA fragment comprising the nucleotide sequence of SEQ ID NO: 4 was isolated.

This construct was microinjected into the pronucleus of zygotes derived from a C57/BL6 x C3H (Central Animal Facility, HHU Düsseldorf) mating, and the surviving zygotes were transferred into the oviduct of pseudopregnant females. After about 18 days the first generation transgenic progeny was born and analyzed for presence of the injected DNA. The transgenic positive mice were crossed with wildtype mice and the resulting progeny was tested for germline transmission.

The inventors of the present invention generated for the first time Fto-overexpressing mice.

### Disclaimer

To provide a comprehensive disclosure without unduly lengthening the specification, the applicant hereby incorporates by reference each of the patents and patent applications referenced above.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. The invention's scope is defined in the following claims and the equivalents thereto. Furthermore, reference signs used in the description and claims do not limit the scope of the invention as claimed.

### References

1. Reed DR et al., BMC Genet. 2008; 9(1): 4.
2. Hager J et al., Nat Genet. 1998; 20(3): 304-8.
3. Hinney A et al., J Clin Endocrinol Metab. 2000; 85(8): 2962-5.
4. Price RA et al., Diabetologia 2001; 44(3): 363-6.
5. Gerken T et al., Science 2007; 318(5855): 1469-72.

## Claims

1. A non-human mammal being partially or completely deficient in expressing a functional gene product encoded by the non-human *Fto* gene.

2. The non-human mammal according to claim 1, **characterized in that** said deficiency is due to genetic alteration based on a homozygous or heterozygous mutation in the non-human *Fto* gene.

3. The non-human mammal according to claim 1 or 2, **characterized in that** said deficiency is due to genetic alteration based on a homozygous or heterozygous deletion in the non-human *Fto* gene.

4. A non-human mammal, said mammal being **characterized in that** the expression level of a non-human Fto protein is increased.

5. The non-human mammal according to claim 4, **characterized in that** the genome of said mammal comprises a transgenic nucleotide sequence encoding for the non-human Fto protein, and wherein said nucleotide sequence is operably linked to a promoter.

6. The non-human mammal according to claim 4 or 5, wherein said promoter exerts its activity in a tissue selected from the group comprising liver, connective tissue, muscle, kidneys, lungs, spleen, pancreas and/or salivary glands.

7. The non-human mammal according to any of claims 4 to 6, wherein said promoter comprises the murine major histocompatibility complex promoter H2-K.

8. The non-human mammal according to any of claims 4 to 7, wherein upstream of the transgenic nucleotide sequence an intron is introduced.

9. The non-human mammal according to any of claims 4 to 8, comprising a transgene construct having the H2-K promoter sequence, the second intron of the β-globin gene from rabbit and the non-human *Fto* cDNA, wherein said construct comprises
a) a nucleic acid molecule having the nucleotide sequence of SEQ ID NO: 4, or a fragment, variant, homologue, or derivative thereof, and/or
b) a nucleic acid molecule having a sequence identity of at least 70, preferably 95 % with any of the nucleic acid molecules of a).

10. The non-human mammal according to any one of the aformentioned claims, which is a rodent.

11. The non-human mammal according to any one of the aformentioned claims, which is a mouse or a rat.

12. A cell, cultured cell, cell line, cell culture and/or tissue culture derived from a non-human mammal according to any of the aforementioned claims.

13. Use of a non-human mammal according to any of claims 1 to 11, and/or a cell, cultured cell, cell line, cell culture and/or tissue culture according to claim 12 as a model for the study of diseases correlated to the expression of human *FTO* protein in a patient.

14. Use according to claim 13, wherein said disease correlated with the expression of *FTO* in a patient is selected from the group comprising obesity, type II diabetes, a cardiovascular disease, and/or dwarfism.

15. Use of a non-human mammal according to any of claims 1 to 11, or of a cell, cultured cell, cell line, cell culture and/or tissue culture according to claim 12 for screening of a compound having therapeutic activity comprising
a) administering a test compound to the non-human mammal or to the cell, cultured cell, cell line, cell culture and/or tissue culture, and
b) evaluating the effect of said compound on said non-human mammal according or cell, cultured cell, cell line, cell culture and/or tissue culture.

16. An antibody, a protease and/or a small molecule drug directed against a FTO protein of human origin and/or an interaction partner thereof and/or directed against an Fto protein of non-human origin and/or an interaction partner thereof.

17. The antibody of claim 16, **characterized in that** this antibody is directed against a human FTO protein and/or against an interaction partner of said human FTO protein, wherein said human FTO protein
a) is encoded by the nucleic acid of SEQ ID NO: 9, or a fragment, variant, homologue or derivative thereof,
b) is encoded by a nucleic acid molecule that is capable of hybridizing to any of the nucleic acid molecules of a) under stringent conditions,
c) is encoded by a nucleic acid molecule having a sequence identity of at least 70, preferably 95 % with any of the nucleic acid molecules of a) or b),
d) is encoded by a nucleic acid molecule that is capable of hybridizing to the complement of any of the nucleic acid molecules of a) - c) under stringent conditions,
e) is encoded by a nucleic acid molecule which comprises, in comparison to the nucleic acid molecule according to a) - d) at least one silent single nucleotide mutation (as allowed by the degeneracy of the genetic code),
f) is encoded by a nucleic acid molecule which, in comparison to the nucleic acid molecule according to a) - e), is code optimized for a given expression host,
g) comprises an amino acid sequence according to SEQ ID NO: 10, or a fragment, variant, homologue or derivative thereof, and/or
h) comprises an amino acid sequence having a sequence identity of at least 70, preferably 95 % with any of the amino acid sequences of g).

18. The protease of claim 16, **characterized in that** this protease is directed against a human FTO protein and/or against an interaction partner of said human FTO protein, wherein said human FTO protein
a) is encoded by the nucleic acid of SEQ ID NO: 9, or a fragment, variant, homologue or derivative thereof,
b) is encoded by a nucleic acid molecule that is capable of hybridizing to any of the nucleic acid molecules of a) under stringent conditions,
c) is encoded by a nucleic acid molecule having a sequence identity of at least 70, preferably 95 % with any of the nucleic acid molecules of a) or b),
d) is encoded by a nucleic acid molecule that is capable of hybridizing to the complement of any of the nucleic acid molecules of a) - c) under stringent conditions,
e) is encoded by a nucleic acid molecule which comprises, in comparison to the nucleic acid molecule according to a) - d) at least one silent single nucleotide mutation (as allowed by the degeneracy of the genetic code),
f) is encoded by a nucleic acid molecule which, in comparison to the nucleic acid molecule according to a) - e), is code optimized for a given expression host,
g) comprises an amino acid sequence according to SEQ ID NO: 10, or a fragment, variant, homologue or derivative thereof, and/or
h) comprises an amino acid sequence having a sequence identity of at least 70, preferably 95 % with any of the amino acid sequences of g).

19. The small molecule drug of claim 16, **characterized in that** this small molecule drug is directed against a human FTO protein and/or against an interaction partner of said human FTO protein, wherein said human FTO protein
a) is encoded by the nucleic acid of SEQ ID NO: 9, or a fragment, variant, homologue or derivative thereof,
b) is encoded by a nucleic acid molecule that is capable of hybridizing to any of the nucleic acid molecules of a) under stringent conditions,
c) is encoded by a nucleic acid molecule having a sequence identity of at least 70, preferably 95 % with any of the nucleic acid molecules of a) or b),
d) is encoded by a nucleic acid molecule that is capable of hybridizing to the complement of any of the nucleic acid molecules of a) - c) under stringent conditions,
e) is encoded by a nucleic acid molecule which comprises, in comparison to the nucleic acid molecule according to a) - d) at least one silent single nucleotide mutation (as allowed by the degeneracy of the genetic code),
f) is encoded by a nucleic acid molecule which, in comparison to the nucleic acid molecule according to a) - e), is code optimized for a given expression host,
g) comprises an amino acid sequence according to SEQ ID NO: 10, or a fragment, variant, homologue or derivative thereof, and/or
h) comprises an amino acid sequence having a sequence identity of at least 70, preferably 95 % with any of the amino acid sequences of g).

20. An antisense construct directed against a nucleic acid related to the *Fto* gene of non-human origin and/or directed against a nucleic acid related to the *FTO* gene of human origin.

21. The antisense construct of claim 20, **characterized in that** this construct is directed against a nucleic acid related to the human *FTO* gene, wherein said human *FTO* gene comprises a nucleic acid sequence of SEQ ID NO: 9, or a fragment, variant, homologue or derivative thereof.

22. The antisense construct of claims 20 to 21, **characterized in that** this construct comprises a nucleotide sequence complementary to
a) the nucleic acid of SEQ ID NO: 9, or a fragment, variant, homologue or derivative thereof ,
b) a nucleic acid molecule having a sequence identity of at least 70, preferably 95 % with any of the nucleic acid molecules of a)
c) a nucleic acid molecule which comprises, in comparison to the nucleic acid molecule according to a) - b) at least one silent single nucleotide mutation (as allowed by the degeneracy of the genetic code), and/or
d) a nucleic acid molecule which, in comparison to the nucleic acid molecule according to a) - c), is code optimized for a given expression host.

23. The antibody of claim 16 or 17, the protease of claim 16 or 18, the SMD of claim 16 or 19 and/or the antisense construct of claims 20 to 22, for use as pharmaceutic and/or a diagnostic composition.

24. The antibody of claim 16 or 17, the protease of claim 16 or 18, the SMD of claim 16 or 19 and/or the antisense construct of claims 20 to 22, for the treatment, prevention and/or diagnosis of a disease correlated with the expression of human *FTO* protein, preferably obesity, type II diabetes, a cardiovascular disease, and/or dwarfism.


SEQUENCE LISTING

<110>  Heinrich-Heine Universität Düsseldorf

<120>  Fto-modified non-human mammal

<130>  UD 40069 / SAM

<160>  10

<170>  PatentIn version 3.3

<210>  1
<211>  3554
<212>  DNA
<213>  Mus musculus

<400>  1
cacggcccac ggtggcgaag gcggctttag tagcagcatg aagcgcgtcc agaccgcgga      60

ggaacgagag cgggaagcta agaaactgag gctccttgag gagcttgaag acacttggct     120

tccttacctg acccccaaag atgatgagtt ctatcagcag tggcagctga aataccctaa     180

actggttttc cgagaggccg gcagcatacc agaggagctg cataaggagg tccccgaggc     240

ctttctcaca ctgcataagc atggctgctt gtttcgggac gtggtgagga tccaaggcaa     300

agatgtgctc accccagtgt ctcgcatcct catcggggac ccaggctgca cctacaagta     360

cttgaacacc agactcttca cggtgccctg gcccgtgaag ggctgcacgg tcaagtacac     420

agaggctgag atcgccgctg catgtcagac cttcctaaag ctcaatgact acctccaggt     480

ggagaccatc caggccttgg aagaactggc tgtcagagag aaggccaatg aagacgctgt     540

gccactgtgc atggcagagt tccccagggc cggcgtgggg ccgtcctgcg atgatgaagt     600

ggaccttaag agcagagcag cctacaacgt gactttgcta aacttcatgg atcctcagaa     660

gatgccctac ttgaaagagg agccctattt cggcatgggg aagatggcgg tgagctggca     720

tcacgatgag aacctggtgg acaggtcagc cgtggcagtg tacagctata gctgcgaagg     780

ctctgaggat gaaagtgagg acgagtccag cttcgaaggc agagatcctg atacttggca     840

tgttggtttt aagatctcat gggacatcga gacaccagga ttaacaatcc ctcttcacca     900

gggagactgc tatttcatgc tggatgacct caatgccacc caccagcact gtgttttggc     960

tggctcacag cctcggttta gttccactca ccgtgtggca gagtgctcaa caggcacctt    1020

ggattatatc ttagaacgct gtcagttggc gctgcagaat gtcctcaatg actcagacga    1080

tggcgacgtc tcgttgaaat cctttgatcc tgcagttttg aaacaaggag aggaaatcca    1140

taatgaggtg gagtttgagt ggctgaggca gttctggttt caaggcagtc gatacaaact    1200

ttgcaccgat tggtggtgtg agcccatgac tcacctggag gggctgtgga agaagatgga    1260

gagcatgaca aatgcggtgc tccgtgaagt taaaagagag gggctcccgg tggaacaaag    1320

gagtgagatt ctgtctgcca tcctggtccc gctcaccgtg cgccagaacc tgaggaagga    1380

gtggcatgcc aggtgccagt cccgagtcgt ccggacttta ccagtacagc agaaaccaga    1440

ctgccggcca tattgggaga aggatgaccc ttccatgcct ctgccctttg acctcacaga    1500

cgtggtttcc gagctcagag gccagctgct ggaagcaaga tcctagagga agcacaagtc    1560

ttaggtggag aaggaaaaga tatccttggc tttcttcctc aaaccttgga caagggtagt 1620
gtagactcct ctcaacccag tggttatagg acctggttcc aattcaaaac agctagagaa 1680
caaagcccac agattctgaa gtgttgaaac gttactgtgt tttagtctga cttggtgtta 1740
aattagagca cccctccctc cccccaaata ttcttcactc accacccccc acacacacct 1800
gaagcccttt tgtctcccaa ggcagcagca gggatgggct aagcctgtga caagcctcac 1860
ctgctctcct ctttggctgg ccacctcaaa gacctggaag taggcctggc catagggtga 1920
cagaactaga gacatccatc ccagcttctc accgtggctt ctagtggtta gaccatccaa 1980
gatggcagcc agtgtctcac cgcggctgca cagatataaa ttgagctcag ttgtcattaa 2040
aactccattc ttcagttgcc ctggcgcatg ctgatgactg ctgacttaga cgacacatac 2100
agatgtttat gtgttccaca tctgtgagcg agccttcctt ctcccagcct tctgtagtgg 2160
ctaaaacatg gggaaaaaca gtgaaaggca gtcaccgccg tcacctcttt cctcctctga 2220
gagggaaggg gccatgagcc ctgtgcactg cacacaggaa gaggaaggag ctacaaggag 2280
acagccaaac cctgtctttg cggggcttcc ctgatccagt gacagaggcc agccctggga 2340
ccactgttct gtggagctag tgtttctcct agaattcccc actcatagat actgacaggt 2400
cgtatggggg cttgaagcac ccagttccca accccgtgac gtcagagcat cagagagagc 2460
atggactctg atttcaaagt gccaggagta agatccaccg cagcactcac tccatgacct 2520
ttgcatgtcc ttgggtctca gttttctcat ctgtatgatt ttgggaggta cccaaaatgc 2580
caactcctaa tgtgaaaatg acgtgatatg acagagctac agatctagag taatttctag 2640
cataccgctg aatatccgat acgttccagt gtcgttacgt gagctgacct ggaagaagcc 2700
gtcttcctcc cccccccccc cccgggtctg cagtcctttg ccgtcttgtg tctcgtggcc 2760
cacatctttc cctagtgacc tgaggccttt tggtggctga aaggttgaca acagttagcc 2820
catgattctc ttgtattggc cgagacctgc aattggaagg acacaacata gttgtggttt 2880
ttgctgcatc caggattcca gttaaggtgc cttcctgcct agaatccacc atagaagctg 2940
cactccacca gccccaaggg cttcctggtg cagggaccca aggctctcag accttgattt 3000
gttccccttt gtctctgagc caggacttcc tcatcagtag agagtgaggt acattctcac 3060
ggggtaaaat ggaacaacct aagtaacggt atagttggct tcatatgtca cagactggcc 3120
cgtctcccca tccccagcca tctggctctg tggtttatta ggccccgtga tatttctttg 3180
ttctatttaa ttgggtgcca gaattaaaaa ttgagatatt ccacataaaa taaaaatgtc 3240
tgcattctct ttagaagcca gaatacctgt gagcccctgg gtctccataa tcgcatggcg 3300
gtggcttggc aatgcctgtg acctttgtgc tctgctgtga ccctgttgct ccctgtcacc 3360
attttgtaaa cctgcgtccg tcccagcagc actttctaga gctgcttgct cgcatcctta 3420
gtagaaagca gtagcgttga gtgctcagtc cctgctcctc ccattggtct aagttcatgg 3480
tctgcttgta ttgctgtgct gtgtgcattt gttcatacac aataaagaat taaacgatga 3540
aaaaaaaaaa aaaa 3554

<210>  2
<211>  502
<212>  PRT
<213>  Mus musculus

<400>  2

Met Lys Arg Val Gln Thr Ala Glu Glu Arg Glu Arg Glu Ala Lys Lys
1               5                   10                  15

Leu Arg Leu Leu Glu Glu Leu Glu Asp Thr Trp Leu Pro Tyr Leu Thr
            20                  25                  30

Pro Lys Asp Asp Glu Phe Tyr Gln Gln Trp Gln Leu Lys Tyr Pro Lys
        35                  40                  45

Leu Val Phe Arg Glu Ala Gly Ser Ile Pro Glu Glu Leu His Lys Glu
    50                  55                  60

Val Pro Glu Ala Phe Leu Thr Leu His Lys His Gly Cys Leu Phe Arg
65                  70                  75                  80

Asp Val Val Arg Ile Gln Gly Lys Asp Val Leu Thr Pro Val Ser Arg
                85                  90                  95

Ile Leu Ile Gly Asp Pro Gly Cys Thr Tyr Lys Tyr Leu Asn Thr Arg
            100                 105                 110

Leu Phe Thr Val Pro Trp Pro Val Lys Gly Cys Thr Val Lys Tyr Thr
        115                 120                 125

Glu Ala Glu Ile Ala Ala Ala Cys Gln Thr Phe Leu Lys Leu Asn Asp
    130                 135                 140

Tyr Leu Gln Val Glu Thr Ile Gln Ala Leu Glu Glu Leu Ala Val Arg
145                 150                 155                 160

Glu Lys Ala Asn Glu Asp Ala Val Pro Leu Cys Met Ala Glu Phe Pro
                165                 170                 175

Arg Ala Gly Val Gly Pro Ser Cys Asp Asp Glu Val Asp Leu Lys Ser
            180                 185                 190

Arg Ala Ala Tyr Asn Val Thr Leu Leu Asn Phe Met Asp Pro Gln Lys
        195                 200                 205

Met Pro Tyr Leu Lys Glu Glu Pro Tyr Phe Gly Met Gly Lys Met Ala
    210                 215                 220

Val Ser Trp His His Asp Glu Asn Leu Val Asp Arg Ser Ala Val Ala
225                 230                 235                 240

Val Tyr Ser Tyr Ser Cys Glu Gly Ser Glu Asp Glu Ser Glu Asp Glu

245                 250                 255

Ser Ser Phe Glu Gly Arg Asp Pro Asp Thr Trp His Val Gly Phe Lys
            260                 265                 270

Ile Ser Trp Asp Ile Glu Thr Pro Gly Leu Thr Ile Pro Leu His Gln
        275                 280                 285

Gly Asp Cys Tyr Phe Met Leu Asp Asp Leu Asn Ala Thr His Gln His
    290                 295                 300

Cys Val Leu Ala Gly Ser Gln Pro Arg Phe Ser Ser Thr His Arg Val
305                 310                 315                 320

Ala Glu Cys Ser Thr Gly Thr Leu Asp Tyr Ile Leu Glu Arg Cys Gln
                325                 330                 335

Leu Ala Leu Gln Asn Val Leu Asn Asp Ser Asp Asp Gly Asp Val Ser
            340                 345                 350

Leu Lys Ser Phe Asp Pro Ala Val Leu Lys Gln Gly Glu Glu Ile His
        355                 360                 365

Asn Glu Val Glu Phe Glu Trp Leu Arg Gln Phe Trp Phe Gln Gly Ser
    370                 375                 380

Arg Tyr Lys Leu Cys Thr Asp Trp Trp Cys Glu Pro Met Thr His Leu
385                 390                 395                 400

Glu Gly Leu Trp Lys Lys Met Glu Ser Met Thr Asn Ala Val Leu Arg
                405                 410                 415

Glu Val Lys Arg Glu Gly Leu Pro Val Glu Gln Arg Ser Glu Ile Leu
            420                 425                 430

Ser Ala Ile Leu Val Pro Leu Thr Val Arg Gln Asn Leu Arg Lys Glu
        435                 440                 445

Trp His Ala Arg Cys Gln Ser Arg Val Val Arg Thr Leu Pro Val Gln
    450                 455                 460

Gln Lys Pro Asp Cys Arg Pro Tyr Trp Glu Lys Asp Asp Pro Ser Met
465                 470                 475                 480

Pro Leu Pro Phe Asp Leu Thr Asp Val Val Ser Glu Leu Arg Gly Gln
                485                 490                 495

Leu Leu Glu Ala Arg Ser
            500

<210>  3
<211>  1980

<212>  DNA
<213>  Mus musculus

<400>  3
aagcttggcc cacaaggagt ggcaatgtta ggagtgtgac tgtgttagag gaagtgtgtc      60

actttgaggt tggcctttga ggtctcctat gctcatgagc tgcccagtgt ggaatgaaag     120

cctcttcctg gctgcatttg gatcaagatg tagaagtctc cccttctcca gcaccatgtc     180

tacctgcaca ctggcatgct tcctgtcatg atggactaaa tccctgaaac tgtaagccag     240

accctattaa atgtctccct ttagaaggaa tgccttggtc atggtgtttc ttcacagcaa     300

caaaaccaaa actaacacac ccagcactta ggatgcaaag gcagatgtat ctctgtgagt     360

tcaagggcag cctgatctac ataatgaatt acagaatagc cagggctgtg cagatagacc     420

ttgtctaaac aagcaaacaa acaaaaccca tagtaaaata agaagaaaag taatggagac     480

agatcctttg tgtagctgcc taaccccaac agcatcccta ggttgatcag gaagaaacct     540

aagagcatca agtccccatg gaaaatgctc acaacatcta ataagagggg ttcagggaga     600

cagggtctgc tgaatcctgg tgaggctggg gggctgctgt ggggatctgc atggggaggt     660

gactggagtt cacacctgtg gtcacacgca ttcattgctc tgtgtgatga gtgactctct     720

tcaggagtcc ccacccatct atgctcttct ttccacttca cttctgtgac tgatttgaaa     780

agatccacag aatcatgaaa tgggtaaatt taatctttcc ccacactact gatcagggat     840

gaaacctcac atcacagtgt gtgctctctg gcatgagaat catctttctc ccagtgtcca     900

cactgcacag gcctgaggaa ctctggggcc aactcaatta tcagaacccg tgtcccacac     960

agctgtgaca ttcatgtccc tcacctcatc agctcaaaag tgtgagtgaa ggtcagggag    1020

ggagggagag ggtcagagat tacataactt ctaccacact agcatcctta ttctgtgtta    1080

tagttggtga cagctcctct cctactgtga ttgagtcact aaagcaactg caccatggaa    1140

gtgcagacac agagaagtcc agtggagaca gggatcagtg ccctgctgtc tgtggtctgc    1200

acggggtctc tcttcagtgg acaagggggt ctcctgtgct gagacaatgt cccagatcca    1260

cagagacaaa ctcaggactc agaatgaaga tccttgtttc aaatacacac acacacacat    1320

acacacacac acacacacac acacacacac acacacacac ctgcagccac agacttttca    1380

tctaggaatt aacacaagga atctgtgtct cagcacaggg ctgagaagac agatcctgag    1440

gggagaggca aagtctacac ttaacagatg agagtcctgc actcaggctt ggcagtgtga    1500

gccgcccatt gcaggtgaac agagcctggt ctctgtgggg tccctgtggg gcttgcagcc    1560

cagcgccttg actttaagga aaagcctctc tctccactgc atccctaagc gcttgtgtcg    1620

ccattgtatt cccggaagag gcttttcttc tagaagactc cagggtctga cttctgaaga    1680

gaagaagaaa gaggaagagt ggaagagagg acacagagag tctggcctgc gggtctctcc    1740

tggtgttttg agagtttctg gatcagaact cggagacgac agcacagggt tcaggcaaag    1800

tcttagtcgc caggcagtga ggtcaggggt ggggaagccc agggctgggg attccccatc    1860

tccacagttt cacttctgca cctaacctgg gtcaggtcct tctgtccgga cactgttgac    1920

gcgcagtcag ctcttacccc cattgggtgg cgcgatcacc aagaaccaat cagtgtcgcc    1980

<210> 4
<211> 5212
<212> DNA
<213> artificial

<220>
<223> transgene construct

<400> 4
aagcttggcc cacaaggagt ggcaatgtta ggagtgtgac tgtgttagag gaagtgtgtc 60

actttgaggt tggcctttga ggtctcctat gctcatgagc tgcccagtgt ggaatgaaag 120

cctcttcctg gctgcatttg gatcaagatg tagaagtctc cccttctcca gcaccatgtc 180

tacctgcaca ctggcatgct tcctgtcatg atggactaaa tccctgaaac tgtaagccag 240

accctattaa atgtctccct ttagaaggaa tgccttggtc atggtgtttc ttcacagcaa 300

caaaaccaaa actaacacac ccagcactta ggatgcaaag gcagatgtat ctctgtgagt 360

tcaagggcag cctgatctac ataatgaatt acagaatagc cagggctgtg cagatagacc 420

ttgtctaaac aagcaaacaa acaaaaccca tagtaaaata agaagaaaag taatggagac 480

agatcctttg tgtagctgcc taaccccaac agcatcccta ggttgatcag gaagaaacct 540

aagagcatca agtccccatg gaaaatgctc acaacatcta ataagagggg ttcagggaga 600

cagggtctgc tgaatcctgg tgaggctggg gggctgctgt ggggatctgc atggggaggt 660

gactggagtt cacacctgtg gtcacacgca ttcattgctc tgtgtgatga gtgactctct 720

tcaggagtcc ccacccatct atgctcttct ttccacttca cttctgtgac tgatttgaaa 780

agatccacag aatcatgaaa tgggtaaatt taatctttcc ccacactact gatcagggat 840

gaaacctcac atcacagtgt gtgctctctg gcatgagaat catctttctc ccagtgtcca 900

cactgcacag gcctgaggaa ctctggggcc aactcaatta tcagaacccg tgtcccacac 960

agctgtgaca ttcatgtccc tcacctcatc agctcaaaag tgtgagtgaa ggtcagggag 1020

ggagggagag ggtcagagat tacataactt ctaccacact agcatcctta ttctgtgtta 1080

tagttggtga cagctcctct cctactgtga ttgagtcact aaagcaactg caccatggaa 1140

gtgcagacac agagaagtcc agtggagaca gggatcagtg ccctgctgtc tgtggtctgc 1200

acggggtctc tcttcagtgg acaagggggt ctcctgtgct gagacaatgt cccagatcca 1260

cagagacaaa ctcaggactc agaatgaaga tccttgtttc aaatacacac acacacacat 1320

acacacacac acacacacac acacacacac acacacacac ctgcagccac agacttttca 1380

tctaggaatt aacacaagga atctgtgtct cagcacaggg ctgagaagac agatcctgag 1440

gggagaggca aagtctacac ttaacagatg agagtcctgc actcaggctt ggcagtgtga 1500

gccgcccatt gcaggtgaac agagcctggt ctctgtgggg tccctgtggg gcttgcagcc 1560

cagcgccttg actttaagga aaagcctctc tctccactgc atccctaagc gcttgtgtcg 1620

ccattgtatt cccggaagag gcttttcttc tagaagactc cagggtctga cttctgaaga 1680

gaagaagaaa gaggaagagt ggaagagagg acacagagag tctggcctgc gggtctctcc 1740

tggtgttttg agagtttctg gatcagaact cggagacgac agcacagggt tcaggcaaag 1800
tcttagtcgc caggcagtga ggtcaggggt ggggaagccc agggctgggg attccccatc 1860
tccacagttt cacttctgca cctaacctgg gtcaggtcct tctgtccgga cactgttgac 1920
gcgcagtcag ctcttacccc cattgggtgg cgcgatcacc aagaaccaat cagtgtcgcc 1980
gagtgaactg cactgtgaca agctgcacgt ggatcctgag aacttcaggg tgagtttggg 2040
gacccttgat tgttctttct ttttcgctat tgtaaaattc atgttatatg gagggggcaa 2100
agttttcagg gtgttgttta gaatgggaag atgtcccttg tatcaccatg gaccctcatg 2160
ataattttgt ttctttcact ttctactctg ttgacaacca ttgtctcctc ttattttctt 2220
ttcattttct gtaacttttt cgttaaactt tagcttgcat ttgtaacgaa tttttaaatt 2280
cacttttgtt tatttgtcag attgtaagta ctttctctaa tcactttttt ttcaaggcaa 2340
tcagggtata ttatattgta cttcagcaca gttttagaga acaattgtta taattaaatg 2400
ataaggtaga atatttctgc atataaattc tggctggcgt ggaaatattc ttattggtag 2460
aaacaactac atcctggtca tcatcctgcc tttctcttta tggttacaat gatatacact 2520
gtttgagatg aggataaaat actctgagtc caaaccgggc ccctctgcta accatgttca 2580
tgccttcttc tttttcctac agctcctggg caacgtgctg gttattgtgc tgtctcatca 2640
ttttggcaaa gaattcccgg gtcgacccac gcgtccgggt ggcgaaggcg gctttagtag 2700
cagcatgaag cgcgtccaga ccgcggagga acgagagcgg gaagctaaga aactgaggct 2760
ccttgaggag cttgaagaca cttggcttcc ttacctgacc cccaaagatg atgagttcta 2820
tcagcagtgg cagctgaaat accctaaact ggttttccga gaggccggca gcataccaga 2880
ggagctgcat aaggaggtcc ccgaggcctt tctcacactg cataagcatg gctgcttgtt 2940
tcgggacgtg gtgaggatcc aaggcaaaga tgtgctcacc ccagtgtctc gcatcctcat 3000
cggggaccca ggctgcacct acaagtactt gaacaccaga ctcttcacgg tgccctggcc 3060
cgtgaagggc tgcacggtca agtacacaga ggctgagatc gccgctgcat gtcagacctt 3120
cctaaagctc aatgactacc tccaggtgga gaccatccag gccttggaag aactggctgt 3180
cagagagaag gccaatgaag acgctgtgcc actgtgcatg gcagagttcc ccagggccgg 3240
cgtggggccg tcctgcgatg atgaagtgga ccttaagagc agagcagcct acaacgtgac 3300
tttgctaaac ttcatggatc ctcagaagat gccctacttg aaagaggagc cctatttcgg 3360
catggggaag atggcggtga gctggcatca cgatgagaac ctggtggaca ggtcagccgt 3420
ggcagtgtac agctatagct gcgaaggctc tgaggatgaa agtgaggacg agtccagctt 3480
cgaaggcaga gatcctgata cttggcatgt tggttttaag atctcatggg acatcgagac 3540
accaggatta acaatccctc ttcaccaggg agactgctat ttcatgctgg atgacctcaa 3600
tgccacccac cagcactgtg ttttggctgg ctcacagcct cggtttagtt ccactcaccg 3660
tgtggcagag tgctcaacag gcaccttgga ttatatctta gaacgctgtc agttggcgct 3720
gcagaatgtc ctcaatgact cagacgatgg cgacgtctcg ttgaaatcct ttgatcctgc 3780
agttttgaaa caaggagagg aaatccataa tgaggtggag tttgagtggc tgaggcagtt 3840

ctggtttcaa ggcagtcgat acaaactttg caccgattgg tggtgtgagc ccatgactca 3900
cctggagggg ctgtggaaga agatggagag catgacaaat gcggtgctcc gtgaagttaa 3960
aagagagggg ctcccggtgg aacaaaggag tgagattctg tctgccatcc tggtcccgct 4020
caccgtgcgc cagaacctga ggaaggagtg gcatgccagg tgccagtccc gagtcgtccg 4080
gactttacca gtacagcaga aaccagactg ccggccatat tgggagaagg atgacccttc 4140
catgcctctg ccctttgacc tcacagacgt ggtttccgag ctcagaggcc agctgctgga 4200
agcaagatcc tagaggaagc acaagtctta ggtggagaag gaaaagatat ccttggcttt 4260
cttcctcaaa ccttggacaa gggtagtgta gactcctctc aacccagtgg ttataggacc 4320
tggttccaat tcaaaacagc tagagaacaa agcccacaga ttctgaagtg ttgaaacgtt 4380
actgtgtttt agtctgactt ggtgttaaat tagagcaccc ctccctcccc ccaaatattc 4440
ttcactcacc accccccaca cacacctgaa gcccttttgt ctcccaaggc agcagcaggg 4500
atgggctaag cctgtgacaa gcctcacctg ctctcctctt tggctggcca cctcaaagac 4560
ctggaagtag gcctggccat agggtgacag aactagagac atccatccca gcttctcacc 4620
gtggcttcta gtggttagac catccaagat ggcagccagt gtctcaccgc ggctgcacag 4680
atataaattg agctcagttg tcattaaaac tccattcttc agttgccctg gcgcatgctg 4740
atgactgctg acttagacga cacatacaga tgtttatgtg ttccacatct gtgagcgagc 4800
cttccttctc ccagccttct gtagtggcta aaacatgggg gaaaacagtg aaaggcagtc 4860
accgccgtca cctctttcct cctctgagag ggaaggggcc atgagccctg tgcactgcac 4920
acaggaagag gaaggagcta caaggagaca gccaaaccct gtctttgcgg ggcttccctg 4980
atccagtgac agaggccagc cctgggacca ctgttctgtg gagctagtgt ttctcctaga 5040
attcactcct caggtgcagg ctgcctatca gaaggtggtg gctggtgtgg ccaatgccct 5100
ggctcacaaa taccactgag atctttttcc ctctgccaaa aattatgggg acatcatgaa 5160
gccccttgag catctgactt ctggctaata aaggaaattt attttcattg ca 5212

<210> 5
<211> 24
<212> DNA
<213> artificial

<220>
<223> Fto Primer for

<400> 5
acccctctcc cccatctaaa tcct 24

<210> 6
<211> 24
<212> DNA
<213> artificial

<220>
<223> Fto Primer rev

<400> 6

aagccaagaa caagtccata cctg                                              24

<210>  7
<211>  21
<212>  DNA
<213>  artificial

<220>
<223>  Neo Primer for

<400>  7
ctgtgctcga cgttgtcact g                                                 21

<210>  8
<211>  21
<212>  DNA
<213>  artificial

<220>
<223>  Neo Primer rev

<400>  8
gatcccctca gaagaactcg t                                                 21

<210>  9
<211>  4294
<212>  DNA
<213>  Homo sapiens

<400>  9
ctacgctctt ccagctgtcg gacctgggaa attctcctgt gctaaatccc gtggcgctcg      60

cgggtgtcgc cgcggtgcat cctgggagtt gtagtttttt ctactcagag ggagaatagc     120

tccagacggg agcaggacgc tgagagaact acatgcagga ggcggggtcc agggcgaggg     180

atctacgcag cttgcggtgg cgaaggcggc tttagtggca gcatgaagcg caccccgact     240

gccgaggaac gagagcgcga agctaagaaa ctgaggcttc ttgaagagct tgaagacact     300

tggctccctt atctgacccc caaagatgat gaattctatc agcagtggca gctgaaatat     360

cctaaactaa ttctccgaga agccagcagt gtatctgagg agctccataa agaggttcaa     420

gaagcctttc tcacactgca caagcatggc tgcttatttc gggacctggt taggatccaa     480

ggcaaagatc tgctcactcc ggtatctcgc atcctcattg gtaatccagg ctgcacctac     540

aagtacctga acaccaggct ctttacggtc ccctggccag tgaaagggtc taatataaaa     600

cacaccgagg ctgaaatagc cgctgcttgt gagaccttcc tcaagctcaa tgactacctg     660

cagatagaaa ccatccaggc tttggaagaa cttgctgcca aagagaaggc taatgaggat     720

gctgtgccat tgtgtatgtc tgcagatttc cccagggttg ggatgggttc atcctacaac     780

ggacaagatg aagtggacat taagagcaga gcagcataca acgtaacttt gctgaatttc     840

atggatcctc agaaaatgcc atacctgaaa gaggaacctt attttggcat ggggaaaatg     900

gcagtgagct ggcatcatga tgaaaatctg gtggacaggt cagcggtggc agtgtacagt     960

tatagctgtg aaggccctga agaggaaagt gaggatgact ctcatctcga aggcagggat    1020

cctgatattt ggcatgttgg ttttaagatc tcatgggaca tagagacacc tggtttggcg    1080

ataccccttc accaaggaga ctgctatttc atgcttgatg atctcaatgc cacccaccaa 1140
cactgtgttt tggccggttc acaacctcgg tttagttcca cccaccgagt ggcagagtgc 1200
tcaacaggaa ccttggatta tattttacaa cgctgtcagt tggctctgca gaatgtctgt 1260
gacgatgtgg acaatgatga tgtctctttg aaatcctttg agcctgcagt tttgaaacaa 1320
ggagaagaaa ttcataatga ggtcgagttt gagtggctga ggcagttttg gtttcaaggc 1380
aatcgataca gaaagtgcac tgactggtgg tgtcaaccca tggctcaact ggaagcactg 1440
tggaagaaga tggagggtgt gacaaatgct gtgcttcatg aagttaaaag agaggggctc 1500
cccgtggaac aaaggaatga aatcttgact gccatccttg cctcgctcac tgcacgccag 1560
aacctgagga gagaatggca tgccaggtgc cagtcacgaa ttgcccgaac attacctgct 1620
gatcagaagc cagaatgtcg gccatactgg gaaaaggatg atgcttcgat gcctctgccg 1680
tttgacctca cagacatcgt ttcagaactc agaggtcagc ttctggaagc aaaaccctag 1740
aaggagcaca agtctcaggc ggaggagaaa aagagatcgg cttttctcct ccaacgttgt 1800
catgggctta agcaagagca gtggagactt ctcttggccc ctagattgta gcacccgggt 1860
cccaatccaa aacagctagg aaatggtgcc catgaagttt taaatgtttt aaaatgaccc 1920
tgtgttatag tctgatttgg tgttaaacag gaccttcttc ccccaaaatt gttcagatta 1980
taaaatgtga gccattcagc ccccaaggtc cagggcaggc gacaggaacg agcccagcgt 2040
gtgacaaagc ctaacctact ttcctctttc ccaagctttt tcagagactc tggagtggac 2100
ccagccctct ggggaaagac agaacttaga gacatcccag ttactcacca cacccatagt 2160
gctgtccaat atggtagcca ctagctagct gtggctactt caatttaaat tcagttttaa 2220
ttttaattaa aaatgcagct cttcagtcgc cctggccaca tttcaagtgc ttaacagcct 2280
catgtggcta gtgactgctg tattggacgg tacagatatg gaacattttc atcatcgaag 2340
aaagtcctat tggacaacac ttctataaaa agtttgagag caggaattct catttccatt 2400
cgtctgtagc ttctatcccc aaaggcaaag aaactaaaag agaaatgact cattgaagat 2460
tggcctcttt cctttctcta agacaaacct aagtaaaagc ctgagctttg agtcctatgc 2520
tcagcacacg ggaaggagat gttaataatt aaaataaagt tgatatcctg tctttaggga 2580
gttcccttga tctcttgaaa gagacacagc cccatttaca ttatttcgtg gatttcacca 2640
gcatagtata gtttttttct gtaagtccct cattcttatg taataacagg tggaactgag 2700
gtttgaagaa cctcagtggc ccatcctgat gacattggag actcaaagag acaagagaga 2760
gtagggttta aaacctgagc tttaagactc ccactagctt cgtgtccttt ggcatgttaa 2820
cgtgcctcag tttcctcatc tgtataatgg ggatatatga aaggcaccag tcctaaggtg 2880
aacattaagt gagatgattc tagttacaga cttagaacaa tttccagcac atagttaaat 2940
atccaggaaa ttctggtact gttatgtgtg ggtgagctga cctggatgta gatgttttcc 3000
tctctcttgc tgacccctcc gccagttttg tcttgtgatg ccattaacac atctctccct 3060
ttctgacctg gctcctgccc attggtgtcc caagaaatcg tgagaatagt tagccccccg 3120
tctccccagc ctgttgcttt ctcgtgtagt tgttcacagt agttgagaag ttgaagagct 3180

tttgcctatt gaaggtgcac tgagaataaa ctctttcctg ccaccagaat tgcagtggtt 3240

cacggcctgc actcattccc atgaatgcag ttaatagcca cagaaatgtc acattaagca 3300

aagcagccag ggtctcatcg tgttgagact cgagtctctc agaccttgga ttcattccct 3360

ggtgtctttg agcctcagtt tcctcattgg taaaagagaa gtgaagcagt gtctcacagg 3420

gtcattacag agattaaatg aaataaatga aataacatag accaggaggg cgtggtgttt 3480

aaaagtcaca gatggggcac cctcgggcca tccagcccag tgttttcttt agcccctatg 3540

atgttcattt tttgttatat cccattaggt gcccatattt aaaaattggg agatttcaca 3600

taaaattaaa aggtctgcat tttctttttt cttttctttt tttttttttt tgagacacag 3660

tctcactctg tcaccaggct agagtgcagt ggcacgatct cagctcactg caacctctgc 3720

ctcccaggtt caagtaattc tcctgcctca gcctcccaag tagctgggac tacaggcacg 3780

tgccaccacg cccagctaat ttttgtattt ttagcagaga tggggtttca ccacattggc 3840

caggatggtc tcgatctcaa cctcgtgatc cacccacctc ggtctcccaa agcgctggga 3900

ttacaggcgt gagccaccgc gccaagccaa ggtctgcatt tttctttaga actcagaaca 3960

cccaatagtc ctaggccccc atcctcgcat ggcagcaagc taaataagca tcttcccact 4020

gcgagttggg gcatgaccca gcctatggtt tgccatactc cctctttttc tccgtttttt 4080

cattaattgt gaacctgacc tgcatcaccc tttcatgtca gtgctctcca aacctgcttg 4140

cttgcacccc tctagtcgaa atattttgtg cttaccccaa tatatgtgtg tgactattga 4200

actctattcg tagactgctt gtactaatgt catttgcatc ataaaatatt catatccaat 4260

aaacatatta aaaggatgag ataagaaacc gaga 4294

<210> 10
<211> 505
<212> PRT
<213> Homo sapiens

<400> 10

Met Lys Arg Thr Pro Thr Ala Glu Glu Arg Glu Arg Glu Ala Lys Lys
1               5                   10                  15

Leu Arg Leu Leu Glu Glu Leu Glu Asp Thr Trp Leu Pro Tyr Leu Thr
            20                  25                  30

Pro Lys Asp Asp Glu Phe Tyr Gln Gln Trp Gln Leu Lys Tyr Pro Lys
        35                  40                  45

Leu Ile Leu Arg Glu Ala Ser Ser Val Ser Glu Glu Leu His Lys Glu
    50                  55                  60

Val Gln Glu Ala Phe Leu Thr Leu His Lys His Gly Cys Leu Phe Arg
65                  70                  75                  80

Asp Leu Val Arg Ile Gln Gly Lys Asp Leu Leu Thr Pro Val Ser Arg
                85                  90                  95

Ile Leu Ile Gly Asn Pro Gly Cys Thr Tyr Lys Tyr Leu Asn Thr Arg
            100                 105                 110

Leu Phe Thr Val Pro Trp Pro Val Lys Gly Ser Asn Ile Lys His Thr
        115                 120                 125

Glu Ala Glu Ile Ala Ala Ala Cys Glu Thr Phe Leu Lys Leu Asn Asp
    130                 135                 140

Tyr Leu Gln Ile Glu Thr Ile Gln Ala Leu Glu Glu Leu Ala Ala Lys
145                 150                 155                 160

Glu Lys Ala Asn Glu Asp Ala Val Pro Leu Cys Met Ser Ala Asp Phe
                165                 170                 175

Pro Arg Val Gly Met Gly Ser Ser Tyr Asn Gly Gln Asp Glu Val Asp
            180                 185                 190

Ile Lys Ser Arg Ala Ala Tyr Asn Val Thr Leu Leu Asn Phe Met Asp
        195                 200                 205

Pro Gln Lys Met Pro Tyr Leu Lys Glu Glu Pro Tyr Phe Gly Met Gly
    210                 215                 220

Lys Met Ala Val Ser Trp His His Asp Glu Asn Leu Val Asp Arg Ser
225                 230                 235                 240

Ala Val Ala Val Tyr Ser Tyr Ser Cys Glu Gly Pro Glu Glu Glu Ser
                245                 250                 255

Glu Asp Asp Ser His Leu Glu Gly Arg Asp Pro Asp Ile Trp His Val
            260                 265                 270

Gly Phe Lys Ile Ser Trp Asp Ile Glu Thr Pro Gly Leu Ala Ile Pro
        275                 280                 285

Leu His Gln Gly Asp Cys Tyr Phe Met Leu Asp Asp Leu Asn Ala Thr
    290                 295                 300

His Gln His Cys Val Leu Ala Gly Ser Gln Pro Arg Phe Ser Ser Thr
305                 310                 315                 320

His Arg Val Ala Glu Cys Ser Thr Gly Thr Leu Asp Tyr Ile Leu Gln
                325                 330                 335

Arg Cys Gln Leu Ala Leu Gln Asn Val Cys Asp Asp Val Asp Asn Asp
            340                 345                 350

Asp Val Ser Leu Lys Ser Phe Glu Pro Ala Val Leu Lys Gln Gly Glu
        355                 360                 365

Glu Ile His Asn Glu Val Glu Phe Glu Trp Leu Arg Gln Phe Trp Phe
    370                 375                 380

Gln Gly Asn Arg Tyr Arg Lys Cys Thr Asp Trp Trp Cys Gln Pro Met
385                 390                 395                 400

Ala Gln Leu Glu Ala Leu Trp Lys Lys Met Glu Gly Val Thr Asn Ala
                405                 410                 415

Val Leu His Glu Val Lys Arg Glu Gly Leu Pro Val Glu Gln Arg Asn
            420                 425                 430

Glu Ile Leu Thr Ala Ile Leu Ala Ser Leu Thr Ala Arg Gln Asn Leu
        435                 440                 445

Arg Arg Glu Trp His Ala Arg Cys Gln Ser Arg Ile Ala Arg Thr Leu
    450                 455                 460

Pro Ala Asp Gln Lys Pro Glu Cys Arg Pro Tyr Trp Glu Lys Asp Asp
465                 470                 475                 480

Ala Ser Met Pro Leu Pro Phe Asp Leu Thr Asp Ile Val Ser Glu Leu
                485                 490                 495

Arg Gly Gln Leu Leu Glu Ala Lys Pro
            500                 505